(19) Europäisches Patentamt
European Patent Office
Office européen des brevets

(11) EP 4 361 122 A1

(12) EUROPEAN PATENT APPLICATION
published in accordance with Art. 153(4) EPC

(43) Date of publication:
01.05.2024 Bulletin 2024/18

(21) Application number: 22828321.4

(22) Date of filing: 16.06.2022

(51) International Patent Classification (IPC):
C07C 2/84 (2006.01)   B01J 23/02 (2006.01)
B01J 23/08 (2006.01)   B01J 23/10 (2006.01)
B01J 23/26 (2006.01)   B01J 23/755 (2006.01)
B01J 23/83 (2006.01)   B01J 23/86 (2006.01)
C07B 61/00 (2006.01)   C07C 2/80 (2006.01)
C07C 11/04 (2006.01)   C25B 3/03 (2021.01)
C25B 5/00 (2006.01)   C25B 9/00 (2021.01)
C25B 9/23 (2021.01)   C25B 11/052 (2021.01)
C25B 11/091 (2021.01)   H01M 8/00 (2016.01)
H01M 8/02 (2016.01)

(52) Cooperative Patent Classification (CPC):
B01J 23/02; B01J 23/08; B01J 23/10; B01J 23/26;
B01J 23/755; B01J 23/83; B01J 23/86;
C07B 61/00; C07C 2/80; C07C 2/84; C07C 11/04;
C25B 3/03; C25B 5/00; C25B 9/00; C25B 9/23;

(Cont.)

(86) International application number:
PCT/JP2022/024127

(87) International publication number:
WO 2022/270404 (29.12.2022 Gazette 2022/52)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 25.06.2021 JP 2021105826

(71) Applicant: Niterra Co., Ltd.
Nagoya-shi, Aichi 461-0005 (JP)

(72) Inventors:
• WATANABE Shuntaro
Nagoya-shi, Aichi 461-0005 (JP)
• NAKAMURA Yosuke
Nagoya-shi, Aichi 461-0005 (JP)
• KOZUKA Hisashi
Nagoya-shi, Aichi 461-0005 (JP)

(74) Representative: Grünecker Patent- und
Rechtsanwälte
PartG mbB
Leopoldstraße 4
80802 München (DE)

(54) ETHYLENE PRODUCTION APPARATUS AND ETHYLENE PRODUCTION METHOD

(57) An ethylene production apparatus for producing ethylene from a methane-containing gas includes a ceramic membrane having at least one of oxide ion conductivity and proton conductivity; a first catalyst layer that is disposed on a first face of the ceramic membrane and includes a catalyst for accelerating oxidative-coupling-of-methane reaction; a methane supply unit that supplies the methane-containing gas to a space located on the same side as the first face of the ceramic membrane; and a charge transfer unit that transfers an electron from the first face to a second face of the ceramic membrane or transfers a hole from the second face to the first face. The ethylene production apparatus produces ethylene in the first catalyst layer upon supply of the methane-containing gas from the methane supply unit.

EP 4 361 122 A1

FIG. 1

METHANE SUPPLY UNIT 30

101

$CH_4$ $C_2H_4$ 24

22

21 $O^{2-}$

20 40 43 42

$O_2$ 26

OXYGEN SUPPLY UNIT 32

(52) Cooperative Patent Classification (CPC): (Cont.)
C25B 11/052; C25B 11/091; H01M 8/00;
H01M 8/02

**Description**

Technical Field

The present disclosure relates to an ethylene production apparatus and an ethylene production method.

Background Art

As known in the art, ethylene is conventionally produced by, for example, thermally cracking a higher hydrocarbon, such as naphtha, followed by fractionation, or thermally cracking ethane derived from natural gas. Other alternative ethylene production methods are suggested in which methane is converted to ethylene by oxidative-coupling-of-methane reaction using a catalyst (see, for example, Patent Literature 1 and Non Patent Literature 1).

Citation List

Patent Literature

PTL 1: JP H5-238961 A

Non Patent Literature

NPL 1: Brittany Lancaster Farrell et al., ACS Catal. 2016, 6, 4340-4346

Summary of Invention

Technical Problem

Ethylene production by a method using an oxidative-coupling-of-methane catalyst advantageously involves less energies than ethylene production by hydrocarbon pyrolysis. From the point of view of industrialization, further enhancements in efficiency are desired in the production of ethylene with an oxidative-coupling-of-methane catalyst.
Solution to Problem
The present disclosure can be implemented as the following aspects.

(1) An aspect of the present disclosure provides an ethylene production apparatus for producing ethylene from a methane-containing gas. The ethylene production apparatus includes a ceramic membrane having at least one of oxide ion conductivity and proton conductivity; a first catalyst layer that is disposed on a first face of the ceramic membrane and includes a catalyst for accelerating oxidative-coupling-of-methane reaction producing ethylene from methane; a methane supply unit that supplies the methane-containing gas to a space that is one of two spaces separated from each other by the ceramic membrane and is located on the same side as the first face of the ceramic membrane; and a charge transfer unit that transfers an electron from the first face to a second face of the ceramic membrane or transfers a hole from the second face to the first face of the ceramic membrane. The ethylene production apparatus produces ethylene in the first catalyst layer upon supply of the methane-containing gas from the methane supply unit to the space on the same side as the first face.
In the ethylene production apparatus according to this aspect, the oxidative-coupling-of-methane reaction can proceed in the first catalyst layer while oxide ions and protons are continuously transferred through the ceramic membrane, and electrons and holes are continuously transferred between the first face and the second face of the ceramic membrane through the charge transfer unit. Thus, the progress of the oxidative-coupling-of-methane reaction can be accelerated, and the efficiency of the whole reaction that produces ethylene from methane can be enhanced.
(2) The ethylene production apparatus according to the above aspect may be such that the ethylene production apparatus further includes an oxygen supply unit that supplies an oxygen-containing gas which contains oxygen to a space located on the same side as the second face of the ceramic membrane; and the charge transfer unit includes an external circuit that connects the first face and the second face of the ceramic membrane to each other, and the charge transfer unit transfers an electron from the first face to the second face of the ceramic membrane through the external circuit by utilizing an electric motive force stemming from an electric potential difference between the first face of the ceramic membrane and the second face of the ceramic membrane. The ethylene production apparatus with the above configuration can attain enhanced efficiency in ethylene production and can provide electric power along with the ethylene production.
(3) The ethylene production apparatus according to the above aspect may be such that the ceramic membrane has oxide ion conductivity; and the ethylene production apparatus further includes a second catalyst layer that is disposed on the second face of the ceramic membrane and includes a catalyst for accelerating a reaction producing an oxide ion from oxygen. According to the above configuration, the ethylene production efficiency can be further enhanced by the addition of the second catalyst layer.

(4) The ethylene production apparatus according to the above aspect may be such that the ceramic membrane has proton conductivity; and the ethylene production apparatus further includes a second catalyst layer that is disposed on the second face of the ceramic membrane and includes a catalyst for accelerating a reaction producing water from oxygen and protons. According to the above configuration, the ethylene production efficiency can be further enhanced by the addition of the second catalyst layer.

(5) The ethylene production apparatus according to the above aspect may be such that the ceramic membrane has proton conductivity; the ethylene production apparatus further includes an inert gas supply unit that supplies an inert gas to a space located on the same side as the second face of the ceramic membrane, or a decompression unit that reduces the pressure in a space located on the same side as the second face of the ceramic membrane, and a second catalyst layer that is disposed on the second face of the ceramic membrane and includes a catalyst for accelerating a reaction producing hydrogen from protons; and the charge transfer unit includes an external circuit that connects the first face and the second face of the ceramic membrane to each other, and the charge transfer unit transfers an electron from the first face to the second face of the ceramic membrane through the external circuit by utilizing an electric motive force stemming from an electric potential difference between the first face of the ceramic membrane and the second face of the ceramic membrane. According to the above configuration, the ethylene production efficiency can be further enhanced by the addition of the second catalyst layer, and the ethylene production apparatus can provide electric power along with the ethylene production. Furthermore, the ethylene production apparatus can produce hydrogen along with the ethylene production.

(6) The ethylene production apparatus according to the above aspect may be such that the ceramic membrane has proton conductivity; the ethylene production apparatus further includes a carbon dioxide supply unit that supplies a carbon dioxide-containing gas which contains carbon dioxide to a space located on the same side as the second face of the ceramic membrane, and a second catalyst layer that is disposed on the second face of the ceramic membrane and includes a catalyst for accelerating a reaction producing methane from carbon dioxide and protons; and the charge transfer unit includes an external circuit that connects the first face and the second face of the ceramic membrane to each other, and the charge transfer unit transfers an electron from the first face to the second face of the ceramic membrane through the external circuit by utilizing an electric motive force stemming from an electric potential difference between the first face of the ceramic membrane and the second face of the ceramic membrane. According to the above configuration, the ethylene production efficiency can be further enhanced by the addition of the second catalyst layer, and the ethylene production apparatus can provide electric power along with the ethylene production. Furthermore, the ethylene production apparatus can produce methane along with the ethylene production.

(7) The ethylene production apparatus according to the above aspect may be such that the ceramic membrane has at least one of electron conductivity and hole conductivity in addition to at least one of oxide ion conductivity and proton conductivity; and the charge transfer unit is the ceramic membrane and allows an electron to transfer from the first face to the second face or allows a hole to transfer from the second face to the first face through the inside of the ceramic membrane itself along with occurrence of ethylene on the first face of the ceramic membrane. The above configuration eliminates the need of an external circuit for charge transfer and can thereby simplify the structure of the apparatus.

(8) The ethylene production apparatus according to the above aspect may be such that the ceramic membrane has oxide ion conductivity and at least one of electron conductivity and hole conductivity; and the ethylene production apparatus further includes an oxygen supply unit that supplies an oxygen-containing gas which contains oxygen to a space located on the same side as the second face of the ceramic membrane, and a second catalyst layer that is disposed on the second face of the ceramic membrane and includes a catalyst for accelerating a reaction producing an oxide ion from oxygen. According to the above configuration, the ethylene production efficiency can be further enhanced by the addition of the second catalyst layer.

(9) The ethylene production apparatus according to the above aspect may be such that the ceramic membrane has proton conductivity and at least one of electron conductivity and hole conductivity; and the ethylene production apparatus further includes an oxygen supply unit that supplies an oxygen-containing gas which contains oxygen to a space located on the same side as the second face of the ceramic membrane, and a second catalyst layer that is disposed on the second face of the ceramic membrane and includes a catalyst for accelerating a reaction using oxygen and a proton. According to the above configuration, the ethylene production efficiency can be further enhanced by the addition of the second catalyst layer.

(10) The ethylene production apparatus according to the above aspect may be such that the ceramic membrane has proton conductivity; and the ethylene production apparatus further includes an inert gas supply unit that supplies an inert gas to a space located on the same side as the second face of the ceramic membrane, or a decompression unit that reduces the pressure in a space located on the same side as the second face of the ceramic membrane, and a second catalyst layer that is disposed on the second face of the ceramic membrane and includes a catalyst for accelerating a reaction producing hydrogen from protons. According to the above configuration, the ethylene production efficiency can be further enhanced by the addition of the second catalyst layer, and the ethylene production apparatus can produce hydrogen along with the ethylene production.

(11) The ethylene production apparatus according to the above aspect may be such that the ceramic membrane has proton conductivity; and the ethylene production apparatus further includes a carbon dioxide supply unit that supplies a carbon dioxide-containing gas which contains carbon dioxide to a space located on the same side as the second face of the ceramic membrane, and a second catalyst layer that is disposed on the second face of the ceramic membrane and includes a catalyst for accelerating a reaction producing methane from carbon dioxide and protons. According to the above configuration, the ethylene production efficiency can be further enhanced by the addition of the second catalyst layer, and the ethylene production apparatus can produce methane along with the ethylene production.

(12) The ethylene production apparatus according to the above aspect may be such that the ethylene production apparatus further includes a methane recovery unit that mixes methane produced on the second face of the ceramic membrane to the methane-containing gas being supplied from the methane supply unit to the space on the same side as the first face of the ceramic membrane. According to the above configuration, methane produced on the second catalyst layer along with ethylene production is mixed, by the methane recovery unit, to the methane-containing gas being supplied to the space on the same side as the first face of the ceramic membrane. Thus, the above configuration saves the amount of methane to be supplied from the methane supply unit to the ethylene production apparatus for the production of ethylene, and can enhance the methane use efficiency.

(13) The ethylene production apparatus according to the above aspect may be such that the catalyst in the first catalyst layer has a perovskite structure represented by general formula $(A_{1-x}A'_x)(Zr_{1-y-z}B_yB'_z)O_3$ (wherein A is at least one element selected from alkaline earth metals, A' is at least one element of lanthanum (La) and yttrium (Y), B is at least one element of titanium (Ti) and cerium (Ce), B' is at least one element selected from yttrium (Y), scandium (Sc), ytterbium (Yb), aluminum (Al), indium (In), and neodymium (Nd), and the letters x, y, and z satisfy $0 \leq x \leq 0.4$, $0.3 \leq (1 - z) \leq 1$, $0 \leq y$, and $0 < (1 - y - z)$). The ethylene production apparatus according to the above configuration can attain a further enhancement in ethylene production efficiency.

(14) The ethylene production apparatus according to the above aspect may be such that the charge transfer unit includes an external circuit that connects the first face and the second face of the ceramic membrane to each other, and an external power supply connected to the external circuit, and the charge transfer unit transfers an electron from the first face to the second face of the ceramic membrane through the external circuit by utilizing an electric motive force from the external power supply. According to the above configuration, the ethylene production efficiency can be enhanced.

(15) Another aspect of the present disclosure provides an ethylene production method for producing ethylene from a methane-containing gas. The ethylene production method includes supplying the methane-containing gas to a space that is one of two spaces separated from each other by a ceramic membrane having at least one of oxide ion conductivity and proton conductivity and is located on the same side as a first face of the ceramic membrane, the first face bearing a first catalyst layer including a catalyst for accelerating oxidative-coupling-of-methane reaction producing ethylene from methane; performing charge transfer between the first face and a second face of the ceramic membrane by transferring an electron from the first face to the second face of the ceramic membrane or transferring a hole from the second face to the first face of the ceramic membrane; and producing ethylene from methane in the methane-containing gas on the first catalyst layer.

In the ethylene production method according to this aspect, the oxidative-coupling-of-methane reaction can proceed in the first catalyst layer while oxide ions and protons are continuously transferred through the ceramic membrane, and electrons and holes are continuously transferred between the first face and the second face of the ceramic membrane. Thus, the progress of the oxidative-coupling-of-methane reaction can be accelerated, and the efficiency of the whole reaction that produces ethylene from methane can be enhanced.

The present disclosure can be implemented as various aspects other than those described above and can be implemented as, for example, an ethylene production method.

Brief Description of Drawings

[Fig. 1] Fig. 1 is a view illustrating a schematic configuration of an ethylene production apparatus of a first embodiment.

[Fig. 2] Fig. 2 is a view illustrating a schematic configuration of an ethylene production apparatus of a second embodiment.

[Fig. 3] Fig. 3 is a view illustrating a schematic configuration of an ethylene production apparatus of a third embodiment.

[Fig. 4] Fig. 4 is a view illustrating a schematic configuration of an ethylene production apparatus of a fourth embodiment.

[Fig. 5] Fig. 5 is a view illustrating a schematic configuration of an ethylene production apparatus of a fifth embodiment.

[Fig. 6] Fig. 6 is a view illustrating a schematic configuration of an ethylene production apparatus of a sixth embodiment.

[Fig. 7] Fig. 7 is a view illustrating a schematic configuration of an ethylene production apparatus of a seventh embodiment.

[Fig. 8] Fig. 8 is a view illustrating a schematic configuration of an ethylene production apparatus of an eighth embodiment.

[Fig. 9] Fig. 9 is a view illustrating a schematic configuration of an ethylene production apparatus of a ninth embodiment.

[Fig. 10] Fig. 10 is a view illustrating a schematic configuration of an ethylene production apparatus of a tenth embodiment.

[Fig. 11] Fig. 11 is a view illustrating a schematic configuration of an ethylene production apparatus of an eleventh embodiment.

[Fig. 12] Fig. 12 is a view describing results of studying the performance of ethylene production apparatuses.

[Fig. 13] Fig. 13 is a view illustrating a schematic configuration of a measurement apparatus for measuring a $C_2$ yield.

[Fig. 14] Fig. 14 is a view describing results of studying the performance of oxidative-coupling-of-methane catalysts.

[Fig. 15] Fig. 15 is a view describing results of studying the performance of oxidative-coupling-of-methane catalysts.

Description of Embodiments

A. First embodiment:

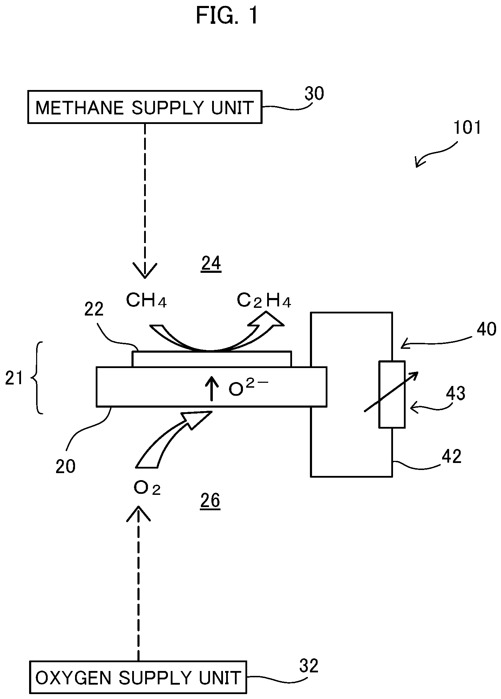

Fig. 1 is a view illustrating a schematic configuration of an ethylene production apparatus 101 according to a first embodiment of the present disclosure. The ethylene production apparatus 101 is an apparatus that produces ethylene from a methane-containing gas. The ethylene production apparatus 101 includes a ceramic membrane 20, a first catalyst layer 22 disposed on a first face of the ceramic membrane 20, a methane supply unit 30, an oxygen supply unit 32, and a charge transfer unit 40. In the following description, the face of the ceramic membrane 20 on which the first catalyst layer 22 is disposed is written as the "first face", and the face on the side different from the face bearing the first catalyst layer 22 is written as the "second face". Furthermore, the ceramic membrane 20 and the first catalyst layer 22 are also collectively referred to as a "membrane structure 21". The ethylene production apparatus 101 further includes a housing (not shown) accommodating two spaces partitioned from each other by a bulkhead. The membrane structure 21 including the ceramic membrane 20 is mounted inside the housing and constitutes at least part of the bulkhead. Of the two spaces in the ethylene production apparatus 101 separated from each other by the membrane structure 21, the space located on the same side as the first face bearing the first catalyst layer 22 is written as a first space 24, and the space located on the same side as the second face of the ceramic membrane 20 is written as a second space 26.

The ceramic membrane 20 is a gas-impermeable dense membrane composed of a ceramic. The ceramic membrane 20 in the present embodiment does not lie on a face of a carrier (a substrate or a support) and is an independent membrane without a carrier. Furthermore, the ceramic membrane 20 contains an oxide ion conductor and exhibits oxide ion conductivity at a temperature and in an atmosphere at or in which the ethylene production apparatus 101 is operated.

For example, the oxide ion conductor contained in the ceramic membrane 20 may be at least one oxide ion conductor selected from stabilized zirconias, partially stabilized zirconias, and ceria-based solid solutions. The stabilized zirconias are zirconias that are stabilized by dissolving one or more oxide dopants into zirconium oxide ($ZrO_2$). Examples of the oxide dopants include yttrium oxide ($Y_2O_3$), scandium oxide ($Sc_2O_3$), ytterbium oxide ($Yb_2O_3$), calcium oxide (CaO), and magnesium oxide (MgO). To attain higher oxide ion conductivity and enhanced stability, the stabilized zirconia is preferably selected from yttria-stabilized zirconia (hereinafter, also written as YSZ) and scandia-stabilized zirconia (hereinafter, also written as ScSZ). Examples of the ceria-based solid solutions include cerium-based composite oxides, such as gadolinium-doped ceria (GDC) and samarium-doped ceria (SDC).

For example, the ceramic membrane 20 may be produced by providing a powder of a raw material, such as the oxide described hereinabove, and forming the powder into a shape by pressing or the like, followed by baking. For example, the thickness of the ceramic membrane 20 may be 1 to 1000 pm. The thickness may be less than 1 $\mu$m as long as the strength and the density of the ceramic membrane 20 are within acceptable ranges. The thickness may exceed 1000 $\mu$m as long as the decrease in oxide ion conduction efficiency due to the large thickness of the ceramic membrane 20 is within an acceptable range.

The first catalyst layer 22 includes an oxidative-coupling-of-methane catalyst that accelerates oxidative-coupling-of-methane reaction producing ethylene from methane. The catalyst present in the first catalyst layer 22 may be any catalyst for accelerating oxidative-coupling-of-methane reaction, and use may be made of a mixture of conventionally known various oxides or a known composite oxide. The first catalyst layer 22 may further have at least one of proton conductivity, electron conductivity, hole conductivity, and oxide ion conductivity. When the first catalyst layer 22 has oxide ion conductivity, the ceramic membrane 20 of the present embodiment has higher oxide ion conductivity than the first catalyst layer 22.

For example, the oxidative-coupling-of-methane catalyst present in the first catalyst layer 22 may be a composite oxide that has a perovskite structure represented by general formula $(A_{1-x}A'_x)(Zr_{1-y-z}B_yB'_z)O_3$ (wherein A is at least one element selected from alkaline earth metals, A' is at least one element of lanthanum (La) and yttrium (Y), B is at least one element of titanium (Ti) and cerium (Ce), B' is at least one element selected from yttrium (Y), scandium (Sc), ytterbium (Yb), aluminum (Al), indium (In), and neodymium (Nd), and the letters x, y, and z satisfy $0 \leq x \leq 0.4$, $0.3 \leq (1 - z) \leq 1$, $0 \leq y$, and $0 < (1 - y - z)$).

For example, the above oxidative-coupling-of-methane catalyst may be produced by a complex polymerization method. A complex polymerization method is a well-known method capable of producing a composite oxide in which component elements are favorably mixed. Specifically, powdery raw materials, such as metal nitrates, are weighed so that the mixing ratio of the metal elements in the powdery raw materials corresponds to the composition of the target composite oxide, and are dissolved into water, and an oxycarboxylic acid, such as citric acid, is added to form a metal oxycarboxylic acid complex. Glycol or the like is added thereto. The mixture is heated to allow the polymerization reaction to proceed, and a polyester polymer gel is thus obtained. The polyester polymer gel is heat treated (baked) to give a composite oxide powder.

The oxide ion conductor or the oxidative-coupling-of-methane catalyst may be produced by a method other than the complex polymerization method described above. Various methods capable of producing composite oxides may be adopted, with examples including solid-phase reaction methods, coprecipitation methods, and sol-gel methods.

For example, the first catalyst layer 22 may be prepared by forming a powder of the oxidative-coupling-of-methane catalyst into a paste using a solvent, and applying the paste onto the ceramic membrane 20, followed by baking. Alternatively, the first catalyst layer 22 may be formed on the ceramic membrane 20 by any of various film-forming methods, such as, for example, a PVD method (a physical vapor deposition method), specifically, for example, a PLD method (a pulse laser deposition method), a dipping method, a thermal spraying method, or a sputtering method.

The first catalyst layer 22 may be constructed using a supported catalyst in which the oxidative-coupling-of-methane catalyst is supported on a porous powder as a carrier. The porous powder used as a carrier may be composed of a material selected from, for example, $CeO_2$, $\gamma$-$Al_2O_3$, zeolite, $SiO_2$, $ZrO_2$, $Na_2WO_4$, $La_2O_3$, $Cs_2SO_4$, $Sm_2O_3$, MgO, SrO, $Y_2O_3$, (La,Sr)$AlO_3$, and $LaAlO_3$. In this case, the particle size of the porous powder is larger than the particle size of the catalyst supported on the porous powder, and may be, for example, 10 nm to 10 pm. The oxidative-coupling-of-methane catalyst may be supported on the carrier by any of various known methods, such as, for example, an impregnation method.

The porous first catalyst layer 22 produced by the above-described method is advantageous in that the catalyst has a large surface area for catalyzing the oxidative-coupling-of-methane reaction and a gas can flow favorably on the catalyst surface.

The thickness of the first catalyst layer 22 may be selected appropriately so as to attain the desired performance in ethylene production depending on factors, such as the degree of catalytic activity of the catalyst present in the first catalyst layer 22, the degree of oxide ion conductivity or electron conductivity of the first catalyst layer 22, and the porosity of the first catalyst layer 22. The thickness of the first catalyst layer 22 may be smaller or larger than the thickness of the ceramic membrane 20.

The first catalyst layer 22 may be composed of a composite catalyst that includes the oxidative-coupling-of-methane catalyst and at least one of a metal catalyst and an oxide catalyst. That is, the first catalyst layer 22 may be composed of a composite catalyst including a combination, such as, for example, a mixture, of the oxidative-coupling-of-methane catalyst and a metal catalyst or an oxide catalyst. Such a first catalyst layer 22 can accelerate the oxidative-coupling-of-methane reaction highly effectively. It is known that metal catalysts and oxide catalysts accelerate a dehydrogenation reaction involved in the oxidative-coupling-of-methane reaction and also promote an oxidation reaction. The acceleration of the oxidation reaction by the use of a metal catalyst and an oxide catalyst can result in the formation of carbon dioxide accompanying ethylene that is the target product. When, however, a metal catalyst or an oxide catalyst is combined with an oxidative-coupling-of-methane catalyst as is the case in the present embodiment, the coupling reaction proceeds preferentially over the oxidation reaction and the formation of ethylene can be further accelerated as a result. For example, the metal catalyst that is used may be a metal selected from palladium (Pd), copper (Cu), iron (Fe), nickel (Ni), platinum (Pt), indium (In), manganese (Mn), ruthenium (Ru), strontium (Sr), zinc (Zn), and lithium (Li). For example, the oxide catalyst that is used may be an oxide selected from $Li_2ASiO_4$ (A is Ca or Sr), $Ce_2(WO_4)_3$, and

CeO$_2$. The composite catalyst may be produced by combining the catalysts described above into a composite using any of various known methods, such as, for example, an impregnation method, optionally together with the use of the carrier described hereinabove.

The methane supply unit 30 is a device that supplies a methane-containing gas to a first space 24 that is one of the two spaces separated from each other by the ceramic membrane 20 in the ethylene production apparatus 101 and is located on the same side as the first face bearing the first catalyst layer 22. For example, the methane-containing gas that is used may be methane-based natural gas or methane gas containing substantially no other components.

The oxygen supply unit 32 is a device that supplies an oxygen-containing gas to a second space 26 that is one of the two spaces separated from each other by the ceramic membrane 20 in the ethylene production apparatus 101 and is located on the same side as the second face different from the face bearing the first catalyst layer 22. For example, the oxygen-containing gas that is used may be air or an oxygen gas containing substantially no other components.

The charge transfer unit 40 transfers an electron from the first face bearing the first catalyst layer 22 to the second face of the ceramic membrane 20. Specifically, the charge transfer unit 40 in the present embodiment includes an external circuit 42 that connects the first face and the second face of the ceramic membrane 20 to each other, and a load unit 43 that is connected to the external circuit 42. The charge transfer unit transfers an electron from the first face to the second face of the ceramic membrane 20 through the external circuit 42 by utilizing an electric motive force stemming from an electric potential difference between the first face and the second face of the ceramic membrane 20. That is, in the present embodiment, the ethylene production apparatus 101 produces ethylene from methane while generating power in the ethylene production apparatus 101, and the electric power can be taken out through the external circuit 42. The load unit 43 may be any device that can take out the electric power generated in the ethylene production apparatus 101 along with ethylene production. The load unit 43 may be any of various electric power consumption devices, and may include an electrical storage device that stores the generated electric power, such as a battery or a capacitor.

The following describes the oxidative-coupling-of-methane (OCM) reaction that is the reaction producing ethylene and takes place in the ethylene production apparatus 101. In the ethylene production apparatus 101, the reaction of the formula (1) below proceeds on the first catalyst layer 22 disposed on the first face of the ceramic membrane 20, and the reaction of the formula (2) below occurs on the second face of the ceramic membrane 20. The reaction of the formula (3) below proceeds in the whole of the ethylene production apparatus 101.

$$2CH_4 + 2O^{2-} \rightarrow C_2H_4 + 2H_2O + 4e^- \cdots \qquad (1)$$

$$O_2 + 4e^- \rightarrow 2O^{2-} \cdots \qquad (2)$$

$$2CH_4 + 2O_2 \rightarrow C_2H_4 + 2H_2O \cdots \qquad (3)$$

Specifically, methane supplied to the first space 24 and oxide ions supplied from the second face of the ceramic membrane 20 through the inside of the ceramic membrane 20 undergo reaction in the first catalyst layer 22 and produce ethylene, water, and electrons (formula (1)). By continuously supplying the methane-containing gas to the first space 24, the gas that is generated in the first catalyst layer 22 and contains ethylene and water is discharged from the first space 24 through a discharge channel (not shown). On the second face of the ceramic membrane 20, the reaction proceeds that generates oxide ions from oxygen supplied to the second space 26 and electrons (formula (2)). In this process, electrons are transferred from the first face to the second face of the ceramic membrane 20 through the external circuit 42. In the ethylene production apparatus 101, ethylene is formed in the first catalyst layer 22 in accordance with the reactions described above when the methane supply unit 30 supplies the methane-containing gas to the first space 24 and the oxygen supply unit 32 supplies the oxygen-containing gas to the second space 26, and ethylene and electric power are extracted from the ethylene production apparatus 101.

While Fig. 1 illustrates a configuration in which the ethylene production apparatus 101 has a single ceramic membrane 20 in the form of a plate, various configurations may be applied to the ethylene production apparatus 101. For example, the ceramic membrane 20 may be cylindrical. In this case, for example, the first catalyst layer 22 may be formed on the inner peripheral surface of the cylindrical ceramic membrane 20 to utilize the inner side of the cylinder as the first space 24, and the outer side of the cylindrical ceramic membrane 20 may be exposed to the outside air to utilize the outer side of the cylinder as the second space 26 in which air as the oxygen-containing gas flows. Furthermore, one end of the cylindrical ceramic membrane 20 may be connected to a channel through which the methane-containing gas is supplied from the methane supply unit 30 to the first space 24, and the other end of the cylindrical ceramic membrane 20 may be connected to a channel for extracting the produced ethylene from the first space 24. Alternatively, the configuration may be such that a plurality of ceramic membranes 20 in the form of plates are stacked on top of one another, the first spaces 24 and the second spaces 26 are provided alternately between the ceramic membranes 20, and the first catalyst layers 22 are formed on the faces of the ceramic membranes 20 exposed to the first spaces 24.

The ethylene production apparatus 101 of the present embodiment configured as described above can continuously perform an operation that supplies oxide ions to the first catalyst layer 22 in which the oxidative-coupling-of-methane reaction proceeds, and an operation that transfers electrons from the first catalyst layer 22 through the charge transfer unit 40 having the external circuit 42. Among the reactions involved in the oxidative-coupling-of-methane reaction, the reaction that produces methyl radicals from methane can be accelerated by the operations. Thus, the efficiency of the whole reaction that produces ethylene from methane can be enhanced. The reaction that produces methyl radicals from methane is further described below.

In the course of the progress of the oxidative-coupling-of-methane reaction, the reaction of the formula (1) that proceeds in the first catalyst layer 22 probably takes place via multiple reactions shown in, for example, the formulas (4) to (6) below. The formula (4) indicates the reaction that produces methyl radicals from methane. The formula (5) describes the reaction in which the methyl radicals are coupled into ethane. The formula (6) indicates the reaction that produces ethylene from ethane.

$$2CH_4 + O^{2-} \rightarrow 2 \cdot CH_3 + H_2O + 2e^- \cdots \qquad (4)$$

$$2 \cdot CH_3 \rightarrow C_2H_6 \cdots \qquad (5)$$

$$C_2H_6 + O^{2-} \rightarrow C_2H_4 + H_2O + 2e^- \cdots \qquad (6)$$

The covalent bond between the carbon atom and the hydrogen atom in methane is very stable with a bond energy of 104 kcal/mol. Thus, it is probable that the rate-determining process in the oxidative-coupling-of-methane reaction is generally the reaction of the formula (4) that produces methyl radicals from methane having a stable covalent bond as described above. Therefore, the acceleration of the methyl radical-forming reaction is important in order to promote the oxidative-coupling-of-methane reaction. The ethylene production apparatus 101 of the present embodiment includes the oxide ion conductive ceramic membrane 20 and is configured to supply methane to the first catalyst layer 22 disposed on the first face of the ceramic membrane 20 and to supply oxygen to the second face of the ceramic membrane 20 as well as to transfer an electron from the first face to the second face of the ceramic membrane 20. Thus, the oxidative-coupling-of-methane reaction is allowed to proceed while oxide ions are supplied to the first catalyst layer 22 through the ceramic membrane 20 and electrons are taken out from the first catalyst layer 22 by the charge transfer unit 40. As a result, the reaction indicated by the formula (4) is accelerated. This acceleration of the rate-determining methyl radical-forming reaction results in acceleration of the whole reaction that produces ethylene from methane, and the ethylene production efficiency can be enhanced.

The ethylene production apparatus 101 of the present embodiment achieves high ethylene production efficiency by providing the first catalyst layer 22 including an oxidative-coupling-of-methane catalyst on the ceramic membrane 20 having relatively high oxide ion conductivity. As described hereinabove, the oxidative-coupling-of-methane catalyst that is used may be, for example, a composite oxide that has a perovskite structure represented by general formula $(A_{1-x}A'_x)(Zr_{1-y-z}B_yB'_z)O_3$ (wherein A is at least one element selected from alkaline earth metals, A' is at least one element of lanthanum (La) and yttrium (Y), B is at least one element of titanium (Ti) and cerium (Ce), B' is at least one element selected from yttrium (Y), scandium (Sc), ytterbium (Yb), aluminum (Al), indium (In), and neodymium (Nd), and the letters x, y, and z satisfy $0 \leq x \leq 0.4$, $0.3 \leq (1 - z) \leq 1$, $0 \leq y$, and $0 < (1 - y - z)$). Such an oxidative-coupling-of-methane catalyst has relatively high activity in accelerating the reaction that produces methyl radicals from methane and therefore has relatively high activity in catalyzing the production of a $C_2$ hydrocarbon, such as ethylene, from methane. Thus, the ethylene production efficiency can be further enhanced.

Furthermore, the ethylene production apparatus 101 of the present embodiment generates electric power along with the reaction that produces ethylene. For example, the generated electric power may be used in the methane supply unit 30 and the oxygen supply unit 32 of the ethylene production apparatus 101 to increase the energy efficiency in the whole of the ethylene production apparatus 101. The generated electric power may be used in devices other than the ethylene production apparatus 101.

B. Second embodiment:

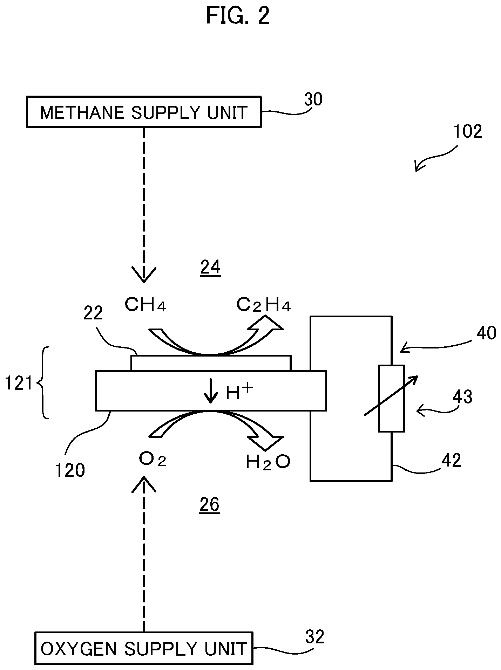

Fig. 2 is a view illustrating a schematic configuration of an ethylene production apparatus 102 of the second embodiment. The ethylene production apparatus 102 has the same structure as the ethylene production apparatus 101 of the first embodiment, except that the ceramic membrane 20 is replaced by a ceramic membrane 120. Hereinafter, the ceramic membrane 120 and the first catalyst layer 22 are also collectively referred to as a "membrane structure 121".

The ceramic membrane 120 present in the ethylene production apparatus 102 is a gas-impermeable dense membrane composed of a ceramic. The membrane is an independent membrane. Furthermore, the ceramic membrane 120 contains a proton conductor and exhibits proton conductivity at a temperature and in an atmosphere at or in which the ethylene production apparatus 102 is operated.

The proton conductor contained in the ceramic membrane 120 may be any of various conventionally known proton conductors. Specific examples include perovskite-type composite oxides, such as $BaZrO_3$-systems, $BaCeO_3$, $SrZrO_3$-systems, and $SrCeO_3$-systems. For example, such a composite oxide may be produced by the complex polymerization method, the solid-phase reaction method, the coprecipitation method, or the sol-gel method described hereinabove.

The following describes the oxidative-coupling-of-methane reaction that is the reaction producing ethylene and takes place in the ethylene production apparatus 102. In the ethylene production apparatus 102, the reaction of the formula (7) below proceeds on the first catalyst layer 22 disposed on the first face of the ceramic membrane 120, and the reaction of the formula (8) below occurs on the second face of the ceramic membrane 120. The reaction of the formula (9) below proceeds in the whole of the ethylene production apparatus 102.

$$2CH_4 \rightarrow C_2H_4 + 4H^+ + 4e^- \cdots \qquad (7)$$

$$O_2 + 4H^+ + 4e^- \rightarrow 2H_2O \cdots \qquad (8)$$

$$2CH_4 + O_2 \rightarrow C_2H_4 + 2H_2O \cdots \qquad (9)$$

Specifically, methane supplied to the first space 24 undergoes reaction in the first catalyst layer 22 and produces ethylene, protons, and electrons (formula (7)). In this process, the electrons formed by the reaction are transferred from the first face to the second face of the ceramic membrane 120 through the external circuit 42, and the protons resulting from the reaction are transferred from the first face to the second face of the ceramic membrane 120 through the inside of the ceramic membrane 120. On the second face of the ceramic membrane 120, the reaction proceeds that generates water from oxygen supplied to the second space 26, the electrons supplied through the external circuit 42, and the protons supplied through the inside of the ceramic membrane 120 (formula (8)). In the ethylene production apparatus 102, ethylene is formed in the first catalyst layer 22 in accordance with the reactions described above when the methane supply unit 30 supplies the methane-containing gas to the first space 24 and the oxygen supply unit 32 supplies the oxygen-containing gas to the second space 26, and ethylene and electric power are extracted from the ethylene production apparatus 102.

According to the above configuration, it is possible to perform continuously an operation that discharges, through the proton conductive ceramic membrane 120, protons from the first catalyst layer 22 in which the oxidative-coupling-of-methane reaction proceeds, and an operation that transfers electrons from the first catalyst layer 22 through the charge transfer unit 40 having the external circuit 42. Among the reactions involved in the oxidative-coupling-of-methane reaction, the reaction that produces methyl radicals from methane can be accelerated by the operations. Thus, the efficiency of the whole reaction that produces ethylene from methane can be enhanced. The reaction that produces methyl radicals from methane is further described below.

In the course of the progress of the oxidative-coupling-of-methane reaction, the reaction of the formula (7) that proceeds in the first catalyst layer 22 probably takes place via multiple reactions shown in, for example, the formulas (10) to (12) below. The formula (10) indicates the reaction that produces methyl radicals from methane. The formula (11) describes the reaction in which the methyl radicals are coupled into ethane, similarly to the formula (5) described hereinabove. The formula (12) indicates the reaction that produces ethylene from ethane.

$$2CH_4 \rightarrow 2 \cdot CH_3 + 2H^+ + 2e^- \cdots \qquad (10)$$

$$2 \cdot CH_3 \rightarrow C_2H_6 \cdots \qquad (11)$$

$$C_2H_6 \rightarrow C_2H_4 + 2H^+ + 2e^- \cdots \qquad (12)$$

As described in the first embodiment, it is probable that the rate-determining process in the oxidative-coupling-of-methane reaction is generally the reaction of the formula (10) that produces methyl radicals from methane having a stable covalent bond. The ethylene production apparatus 102 of the second embodiment includes the proton conductive ceramic membrane 120 and is configured to supply methane to the first catalyst layer 22 disposed on the first face of the ceramic membrane 120 and to supply oxygen to the second face of the ceramic membrane 120 as well as to transfer an electron from the first face to the second face of the ceramic membrane 20. Thus, the oxidative-coupling-of-methane reaction is allowed to proceed while protons are discharged from the first catalyst layer 22 through the ceramic membrane 20 and electrons are taken out from the first catalyst layer 22 by the charge transfer unit 40. As a result, the reaction indicated by the formula (10) is accelerated. This acceleration of the rate-determining methyl radical-forming reaction results in acceleration of the whole reaction that produces ethylene from methane, and the ethylene production efficiency can be enhanced.

C. Third embodiment:

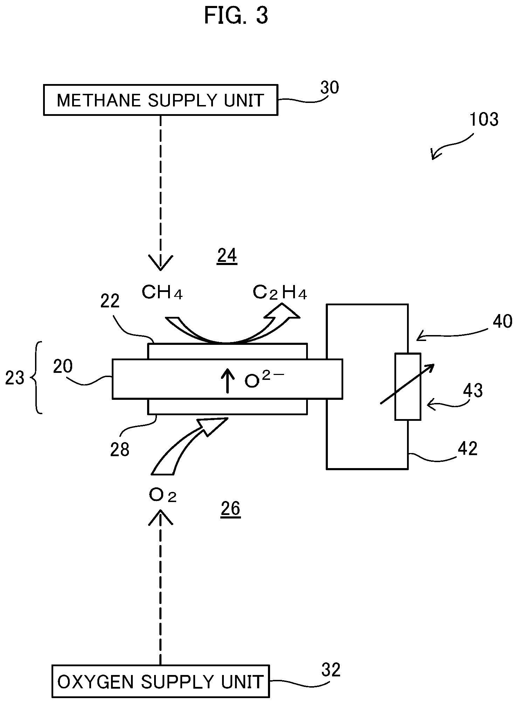

Fig. 3 is a view illustrating a schematic configuration of an ethylene production apparatus 103 of the third embodiment. The ethylene production apparatus 103 has the same structure as the ethylene production apparatus 101 of the first embodiment, except that a second catalyst layer 28 is further disposed on the second face of the ceramic membrane 20. Hereinafter, the ceramic membrane 20, the first catalyst layer 22, and the second catalyst layer 28 are also collectively referred to as a "membrane structure 23".

In the ethylene production apparatus 103 of the third embodiment, reactions similar to those in the ethylene production apparatus 101 of the first embodiment proceed. Specifically, the reactions indicated in the formulas (1) and (4) to (6) proceed in the first catalyst layer 22, and the reaction shown in the formula (2) takes place in the second catalyst layer 28. The reaction indicated in the formula (3) proceeds in the whole of the ethylene production apparatus 103. The second catalyst layer 28 present in the ethylene production apparatus 103 of the third embodiment includes a catalyst for accelerating the reaction indicated in the formula (2). The catalyst present in the second catalyst layer 28 is not particularly limited as long as it is capable of accelerating the reaction of the formula (2). The catalyst present in the second catalyst layer 28 is preferably an oxide having oxide ion-electron mixed conductivity. For example, $La_{0.6}Sr_{0.4}Co_{0.2}Fe_{0.8}O_3$ may be used. The above composite oxide used as the catalyst in the second catalyst layer 28 may be produced by, for example, the complex polymerization method, the solid-phase reaction method, the coprecipitation method, or the sol-gel method described hereinabove. The second catalyst layer 28 may be formed on the ceramic membrane 20 using the catalyst obtained, in the same manner as the first catalyst layer 22.

The above configuration offers the same effects as those of the ethylene production apparatus 101 of the first embodiment and can further accelerate the reaction shown in the formula (2) by the addition of the second catalyst layer 28. As a result, the reaction of the formula (3) that takes place in the whole of the ethylene production apparatus 103 can be accelerated to further enhance the ethylene production efficiency.

D. Fourth embodiment:

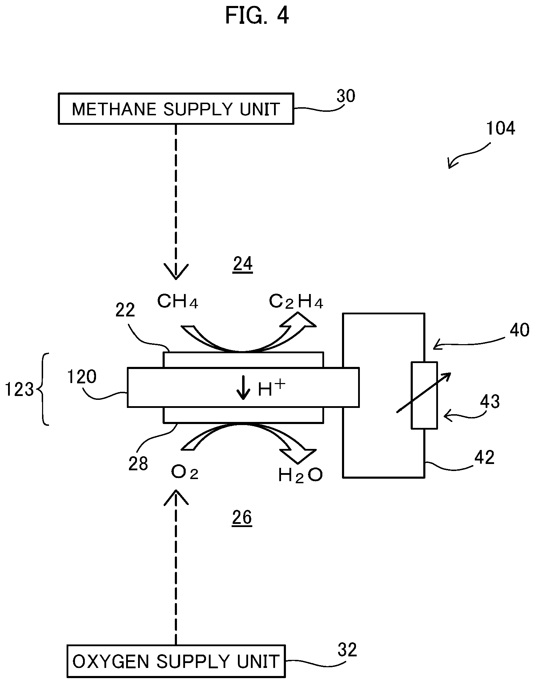

Fig. 4 is a view illustrating a schematic configuration of an ethylene production apparatus 104 of the fourth embodiment. The ethylene production apparatus 104 has the same structure as the ethylene production apparatus 102 of the second embodiment, except that a second catalyst layer 28 is further disposed on the second face of the ceramic membrane 120. Hereinafter, the ceramic membrane 120, the first catalyst layer 22, and the second catalyst layer 28 are also collectively referred to as a "membrane structure 123".

In the ethylene production apparatus 104 of the fourth embodiment, reactions similar to those in the ethylene production apparatus 102 of the second embodiment proceed. Specifically, the reactions indicated in the formulas (7) and (10) to (12) proceed in the first catalyst layer 22, and the reaction shown in the formula (8) takes place in the second catalyst layer 28. The reaction indicated in the formula (9) proceeds in the whole of the ethylene production apparatus 103.

The second catalyst layer 28 present in the ethylene production apparatus 104 of the fourth embodiment includes a catalyst for accelerating the reaction indicated in the formula (8). The catalyst present in the second catalyst layer 28 is not particularly limited as long as it is capable of accelerating the reaction of the formula (8). The catalyst present in the second catalyst layer 28 is preferably an oxide having oxide ion-electron mixed conductivity. For example, $La_{0.6}Sr_{0.4}Co_{0.2}Fe_{0.8}O_3$ may be used. The second catalyst layer 28 in the fourth embodiment may be produced in the same manner as the second catalyst layer 28 in the third embodiment.

The above configuration offers the same effects as those of the ethylene production apparatus 102 of the second embodiment and can further accelerate the reaction shown in the formula (8) by the addition of the second catalyst layer 28. As a result, the reaction of the formula (9) that takes place in the whole of the ethylene production apparatus 103 can be accelerated to further enhance the ethylene production efficiency.

E. Fifth embodiment:

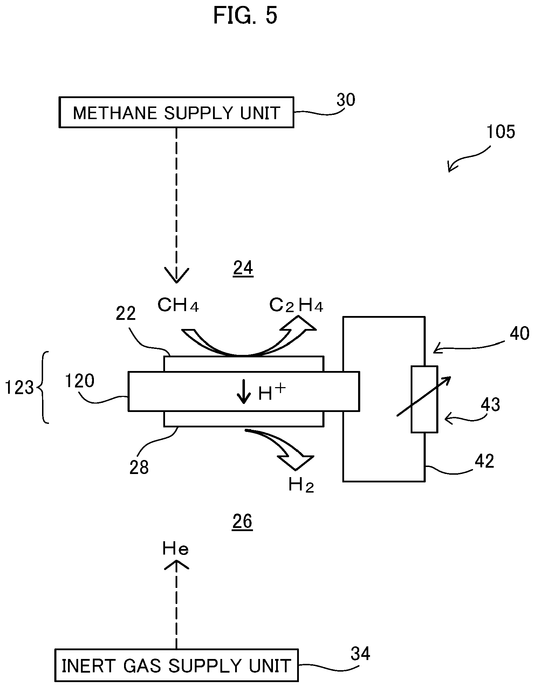

Fig. 5 is a view illustrating a schematic configuration of an ethylene production apparatus 105 of the fifth embodiment. The ethylene production apparatus 105 has the same structure as the ethylene production apparatus 104 of the fourth embodiment, except that the oxygen supply unit 32 that supplies an oxygen-containing gas to the second space 26 is replaced by an inert gas supply unit 34 that supplies an inert gas to the second space 26. The inert gas supplied by the inert gas supply unit 34 may be any gas that does not affect the reaction occurring on the second catalyst layer 28. For example, helium, argon, nitrogen, or the like may be used.

The second catalyst layer 28 present in the ethylene production apparatus 105 includes a catalyst for accelerating the reaction of the formula (14) described later, in place of the reaction of the formula (8) occurring on the second catalyst layer 28 in the fourth embodiment. The catalyst present in the second catalyst layer 28 of the fifth embodiment is not particularly limited as long as it is capable of accelerating the reaction of the formula (14). The catalyst present in the second catalyst layer 28 is preferably an oxide having oxide ion-electron mixed conductivity. For example, $La_{0.6}Sr_{0.4}Co_{0.2}Fe_{0.8}O_3$ may be used. The second catalyst layer 28 in the fifth embodiment may be produced in the same manner as the second catalyst layer 28 in the fourth embodiment.

In the ethylene production apparatus 105, the reaction of the formula (13) below (same as the reaction of the formula (7)) proceeds in the first catalyst layer 22 disposed on the first face of the ceramic membrane 120, and the reaction of the formula (14) below takes place on the second face of the ceramic membrane 120. The reaction of the formula (15) below proceeds in the whole of the ethylene production apparatus 102.

$$2CH_4 \rightarrow C_2H_4 + 4H^+ + 4e^- \cdots \qquad (13)$$

$$2H^+ + 2e^- \rightarrow H_2 \cdots \qquad (14)$$

$$2CH_4 \rightarrow C_2H_4 + 2H_2 \cdots \qquad (15)$$

Specifically, similarly to the reaction of the formula (7) in the second and the fourth embodiments, methane supplied to the first space 24 undergoes reaction in the first catalyst layer 22 and produces ethylene, protons, and electrons (formula (13)). In this process, the electrons formed by the reaction are transferred from the first face to the second face of the ceramic membrane 120 through the external circuit 42, and the protons resulting from the reaction are transferred from the first face to the second face of the ceramic membrane 120 through the inside of the ceramic membrane 120. On the second face of the ceramic membrane 120, the reaction proceeds that generates hydrogen from the electrons supplied through the external circuit 42 and the protons supplied through the inside of the ceramic membrane 120 (formula (14)). The supply of an inert gas to the second space 26 lowers the partial pressure of hydrogen in the second space 26 and allows the progress of the reaction of the formula (14) to be maintained. In the ethylene production apparatus 105, ethylene is formed in the first catalyst layer 22 in accordance with the reactions described above when the methane supply unit 30 supplies the methane-containing gas to the first space 24 and the inert gas supply unit 34 supplies the inert gas to the second space 26, and ethylene, hydrogen, and electric power are extracted from the ethylene production apparatus 105. In the course of the progress of the oxidative-coupling-of-methane reaction in the ethylene production apparatus 105 of the fifth embodiment, the reaction of the formula (13) that proceeds in the first catalyst layer 22 probably takes place, for example, via the multiple reactions shown in the formulas (10) to (12) hereinabove.

Similarly to the case of the ethylene production apparatus 104 of the fourth embodiment, the above configuration can effectively enhance the ethylene production efficiency. Furthermore, the ethylene production apparatus 105 of the fifth embodiment generates hydrogen in the second catalyst layer 28 along with the ethylene formation in the first catalyst layer 22. The hydrogen obtained may be used as, for example, a fuel for combustion reactions or power generation to produce energy, such as thermal energy or electrical energy.

In the ethylene production apparatus 105 of the fifth embodiment illustrated in Fig. 5, the inert gas supply unit 34 may be replaced by a decompression unit that reduces the pressure in the second space 26. For example, the decompression unit may be composed of a decompression pump. This configuration too can lower the partial pressure of hydrogen generated in the second catalyst layer 28 in the second space 26 similarly to the case where the inert gas supply unit 34 is provided. The progress of the reaction of the formula (14) can be thus maintained.

F. Sixth embodiment:

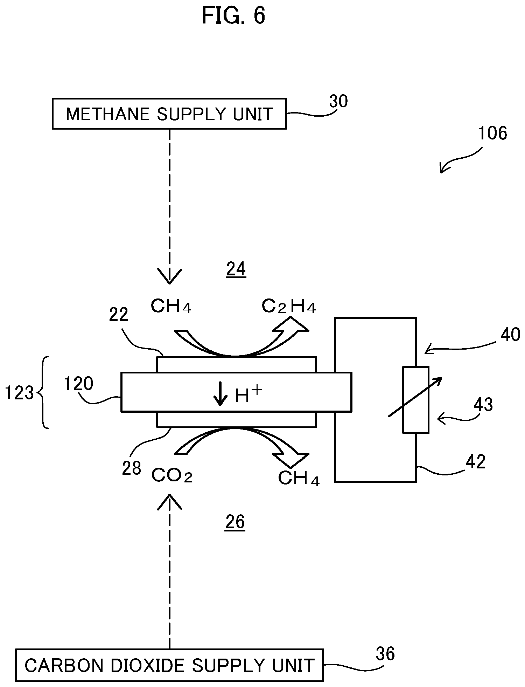

Fig. 6 is a view illustrating a schematic configuration of an ethylene production apparatus 106 of the sixth embodiment. The ethylene production apparatus 106 has the same structure as the ethylene production apparatus 104 of the fourth embodiment, except that the oxygen supply unit 32 that supplies the oxygen-containing gas to the second space 26 is replaced by a carbon dioxide supply unit 36 that supplies a carbon dioxide-containing gas which contains carbon dioxide to the second space 26.

The second catalyst layer 28 present in the ethylene production apparatus 106 includes a catalyst for accelerating the reaction of the formula (17) described later, in place of the reaction of the formula (8) occurring on the second catalyst layer 28 in the fourth embodiment. The catalyst present in the second catalyst layer 28 of the sixth embodiment is not particularly limited as long as it is capable of accelerating the reaction of the formula (17). For example, the catalyst used in the second catalyst layer 28 may be nickel. When nickel is used, the second catalyst layer 28 may be prepared by, for example, forming a powder of nickel oxide into a paste using a solvent, and applying the paste onto the second face of the ceramic membrane 120, followed by baking.

In the ethylene production apparatus 106, the reaction of the formula (16) below (same as the reaction of the formula (7)) proceeds in the first catalyst layer 22 disposed on the first face of the ceramic membrane 120, and the reaction of the formula (17) below takes place on the second face of the ceramic membrane 120. The reaction of the formula (18) below proceeds in the whole of the ethylene production apparatus 106.

$$2CH_4 \rightarrow C_2H_4 + 4H^+ + 4e^- \cdots \qquad (16)$$

$$CO_2 + 8H^+ + 8e^- \rightarrow CH_4 + 2H_2O \cdots \qquad (17)$$

$$3CH_4 + CO_2 \rightarrow 2C_2H_4 + 2H_2O \cdots \qquad (18)$$

Specifically, similarly to the reaction of the formula (7) in the second and the fourth embodiments, methane supplied to the first space 24 undergoes reaction in the first catalyst layer 22 and produces ethylene, protons, and electrons (formula (16)). In this process, the electrons formed by the reaction are transferred from the first face to the second face of the ceramic membrane 120 through the external circuit 42, and the protons resulting from the reaction are transferred from the first face to the second face of the ceramic membrane 120 through the inside of the ceramic membrane 120. On the second face of the ceramic membrane 120, the reaction proceeds that generates methane and water from the carbon dioxide supplied to the second space 26, the electrons supplied through the external circuit 42, and the protons supplied through the inside of the ceramic membrane 120 (formula (17)). In the ethylene production apparatus 106, ethylene is formed in the first catalyst layer 22 in accordance with the reactions described above when the methane supply unit 30 supplies the methane-containing gas to the first space 24 and the carbon dioxide supply unit 36 supplies carbon dioxide to the second space 26, and ethylene, methane, and electric power are extracted from the ethylene production apparatus 105. In the course of the progress of the oxidative-coupling-of-methane reaction in the ethylene production apparatus 106 of the sixth embodiment, the reaction of the formula (16) that proceeds in the first catalyst layer 22 probably takes place, for example, via the multiple reactions shown in the formulas (10) to (12) hereinabove.

Similarly to the case of the ethylene production apparatus 104 of the fourth embodiment, the above configuration can effectively enhance the ethylene production efficiency. Furthermore, the ethylene production apparatus 106 of the sixth embodiment generates methane in the second catalyst layer 28 along with the ethylene formation in the first catalyst layer 22. The methane obtained may be used as, for example, a fuel for combustion reactions to produce energy, or may be used as a material for synthesis of other compounds.

G. Seventh embodiment:

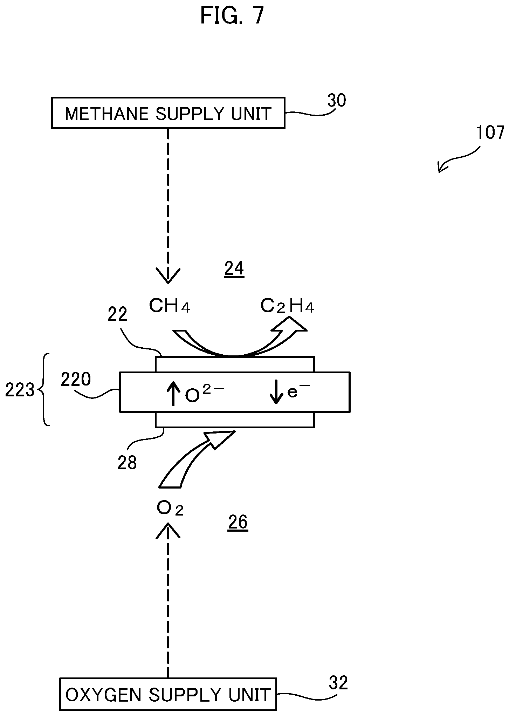

Fig. 7 is a view illustrating a schematic configuration of an ethylene production apparatus 107 of the seventh embodiment. The ethylene production apparatus 107 has the same structure as the ethylene production apparatus 103 of the third embodiment, except that the ceramic membrane 20 is replaced by a ceramic membrane 220, and the apparatus does not include the charge transfer unit 40 having the external circuit 42. Hereinafter, the ceramic membrane 220, the first catalyst layer 22, and the second catalyst layer 28 are also collectively referred to as a "membrane structure 223".

The ceramic membrane 220 present in the ethylene production apparatus 107 is a gas-impermeable dense membrane composed of a ceramic. The membrane is an independent membrane. Furthermore, the ceramic membrane 220 contains an oxide ion conductor and at least one of an electron conductor and a hole conductor, and exhibits oxide ion conductivity and at least one of electron conductivity and hole conductivity at a temperature and in an atmosphere at or in which the ethylene production apparatus 107 is operated. In the following description, electron conductivity and hole conductivity are also written collectively as "electron conductivity", and an electron conductor and a hole conductor are also written collectively as an "electron conductor".

For example, the ceramic membrane 220 may be formed of a mixture of an oxide ion conductor and an electron conductor. Alternatively, the ceramic membrane 220 may be formed of a composite oxide having both oxide ion conductivity and electron conductivity (hereinafter, such an oxide is also written as a first mixed-conductivity oxide). Alternatively, the ceramic membrane 220 may be formed of a mixture of at least one of an oxide ion conductor and an electron conductor, and a first mixed-conductivity oxide.

For example, the oxide ion conductor contained in the ceramic membrane 220 may be similar to the oxide ion conductor used in the ceramic membrane 20 of the first embodiment or the third embodiment.

For example, the electron conductor contained in the ceramic membrane 220 may be an electron conductor selected from oxide electron conductors having a perovskite structure, oxide electron conductors having a spinel-type crystal structure, and metal materials, such as noble metals. Examples of the oxide electron conductors having a perovskite structure include LSM oxides obtained by doping $LaMnO_3$ compounds with Sr at La sites, and composite oxides, such as $SrTiO_3$. Alternatively, use may be made of at least one electron conductor selected from electron conductors represented by the formula (19) below and electron conductors represented by the formula (20) below. The alkaline earth metal M in the formula (19) is preferably strontium (Sr) or calcium (Ca).

$$La_{1-x}M_xCrO_3 \cdots \qquad (19)$$

(In the formula, M is an element selected from alkaline earth metals except magnesium (Mg), and $0 \leq x \leq 0.3$.)

$$LaCr_{1-x}Mg_xO_3 \cdots \qquad (20)$$

(In the formula, $0 \leq x \leq 0.3$.)

Examples of the first mixed-conductivity oxides contained in the ceramic membrane 220 include LSGF oxides having a perovskite structure that are obtained by doping $LaGaO_3$ compounds with Sr at La sites and with Fe at Ga sites, and BSCF oxides having a perovskite structure that are obtained by doping $SrCoO_3$ compounds with Ba at Sr sites and with Fe at Co sites. Other materials usable as the first mixed-conductivity oxides include oxides having a layered perovskite structure, oxides having a fluorite-type structure, oxides having an oxyapatite structure, and oxides having a melilite structure.

For example, the above composite oxides for use as the electron conductors or the first mixed-conductivity oxides may be produced by the complex polymerization method, the solid-phase reaction method, the coprecipitation method, or the sol-gel method described hereinabove. The oxide ion conductor and the electron conductor may be obtained as a mixed powder, or the first mixed-conductivity oxide may be obtained as a powder, and the powder may be shaped by pressing or the like and baked to give a ceramic membrane 220.

In the ethylene production apparatus 107 of the seventh embodiment, reactions similar to those in the ethylene production apparatus 103 of the third embodiment proceed. Specifically, the reactions indicated in the formulas (1) and (4) to (6) proceed in the first catalyst layer 22, and the reaction shown in the formula (2) takes place in the second catalyst layer 28. The reaction indicated in the formula (3) proceeds in the whole of the ethylene production apparatus 107.

The ethylene production apparatus 107 of the seventh embodiment differs from the ethylene production apparatus 103 of the third embodiment in that the electrons generated in the reaction of the formula (1) are transferred to the second catalyst layer 28 through the inside of the ceramic membrane 220 and participate in the reaction of the formula (2). Thus, the ceramic membrane 220 in the seventh embodiment functions as a charge transfer unit.

Similarly to the case of the ethylene production apparatus 103 of the third embodiment, the above configuration can effectively enhance the ethylene production efficiency. Furthermore, the ceramic membrane 220 constructs a charge transfer unit and eliminates the need of the external circuit 42, thereby simplifying the configuration of the apparatus.

H. Eighth embodiment:

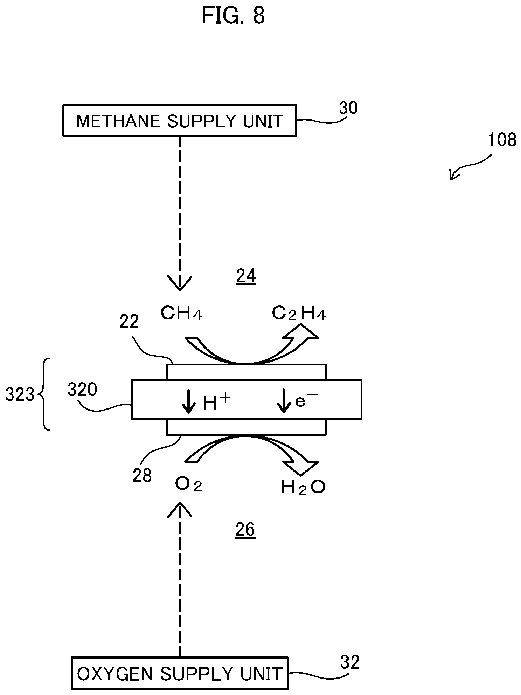

Fig. 8 is a view illustrating a schematic configuration of an ethylene production apparatus 108 of the eighth embodiment. The ethylene production apparatus 108 has the same structure as the ethylene production apparatus 104 of the fourth embodiment, except that the ceramic membrane 120 is replaced by a ceramic membrane 320, and the apparatus does not include the charge transfer unit 40 having the external circuit 42. Hereinafter, the ceramic membrane 320, the first catalyst layer 22, and the second catalyst layer 28 are also collectively referred to as a "membrane structure 323".

The ceramic membrane 320 present in the ethylene production apparatus 108 is a gas-impermeable dense membrane composed of a ceramic. The membrane is an independent membrane. Furthermore, the ceramic membrane 320 exhibits proton conductivity and electron conductivity at a temperature and in an atmosphere at or in which the ethylene production apparatus 108 is operated.

For example, the ceramic membrane 320 may be formed of a mixture of a proton conductor and an electron conductor. Alternatively, the ceramic membrane 320 may be formed of a composite oxide having both proton conductivity and electron conductivity (hereinafter, such an oxide is also written as a second mixed-conductivity oxide). Alternatively, the ceramic membrane 320 may be formed of a mixture of at least one of a proton conductor and an electron conductor, and a second mixed-conductivity oxide.

For example, the proton conductor contained in the ceramic membrane 320 may be similar to the proton conductor used in the ceramic membrane 120 of the second and the fourth to the sixth embodiments. The electron conductor contained in the ceramic membrane 320 may be similar to the electron conductor used in the ceramic membrane 220 of the seventh embodiment. The proton conductor and the electron conductor may be obtained as a mixed powder, or the second mixed-conductivity oxide may be obtained as a powder, and the powder may be shaped by pressing or the like and baked to give a ceramic membrane 320.

In the ethylene production apparatus 108 of the eighth embodiment, reactions similar to those in the ethylene production apparatus 104 of the fourth embodiment proceed. Specifically, the reactions indicated in the formulas (7) and (10) to (12) proceed in the first catalyst layer 22, and the reaction shown in the formula (8) takes place in the second catalyst layer 28. The reaction indicated in the formula (9) proceeds in the whole of the ethylene production apparatus 108.

The ethylene production apparatus 108 of the eighth embodiment differs from the ethylene production apparatus 104 of the fourth embodiment in that the electrons generated in the reaction of the formula (7) are transferred to the second catalyst layer 28 through the inside of the ceramic membrane 320 and participate in the reaction of the formula (8). Thus, the ceramic membrane 320 in the eighth embodiment functions as a charge transfer unit.

Similarly to the case of the ethylene production apparatus 104 of the fourth embodiment, the above configuration can effectively enhance the ethylene production efficiency. Furthermore, the ceramic membrane 320 constructs a charge transfer unit and eliminates the need of the external circuit 42, thereby simplifying the configuration of the apparatus.

I. Ninth embodiment:

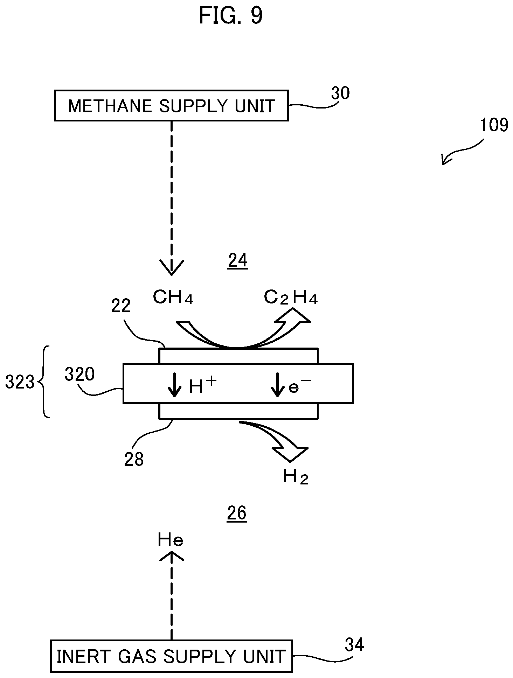

Fig. 9 is a view illustrating a schematic configuration of an ethylene production apparatus 109 of the ninth embodiment. The ethylene production apparatus 109 has the same structure as the ethylene production apparatus 105 of the fifth embodiment, except that the ceramic membrane 120 is replaced by a ceramic membrane 320, and the apparatus does not include the charge transfer unit 40 having the external circuit 42. The ceramic membrane 320 formed in the ethylene production apparatus 109 of the ninth embodiment is similar to the ceramic membrane 320 in the eighth embodiment.

In the ethylene production apparatus 109 of the ninth embodiment, reactions similar to those in the ethylene production apparatus 105 of the fifth embodiment proceed. Specifically, the reactions indicated in the formula (13) (same as the reaction of the formula (7)) and the formulas (10) to (12) proceed in the first catalyst layer 22, and the reaction shown in the formula (14) takes place in the second catalyst layer 28. The reaction indicated in the formula (15) proceeds in the whole of the ethylene production apparatus 108.

The ethylene production apparatus 109 of the ninth embodiment differs from the ethylene production apparatus 105 of the fifth embodiment in that the electrons generated in the reaction of the formula (13) are transferred to the second catalyst layer 28 through the inside of the ceramic membrane 320 and participate in the reaction of the formula (14). Thus, the ceramic membrane 320 in the ninth embodiment functions as a charge transfer unit.

Similarly to the case of the ethylene production apparatus 105 of the fifth embodiment, the above configuration can effectively enhance the ethylene production efficiency. Furthermore, hydrogen is generated in the second catalyst layer 28 along with the ethylene formation in the first catalyst layer 22. The hydrogen obtained may be used in, for example, energy production. Furthermore, the ceramic membrane 320 constructs a charge transfer unit and eliminates the need of the external circuit 42, thereby simplifying the configuration of the apparatus.

In the ethylene production apparatus 109 of the fifth embodiment illustrated in Fig. 9, the inert gas supply unit 34 may be replaced by a decompression unit that reduces the pressure in the second space 26. For example, the decompression unit may be composed of a decompression pump. This configuration too can lower the partial pressure of hydrogen generated in the second catalyst layer 28 in the second space 26 similarly to the case where the inert gas supply unit 34 is provided. The progress of the reaction of the formula (14) can be thus maintained.

J. Tenth embodiment:

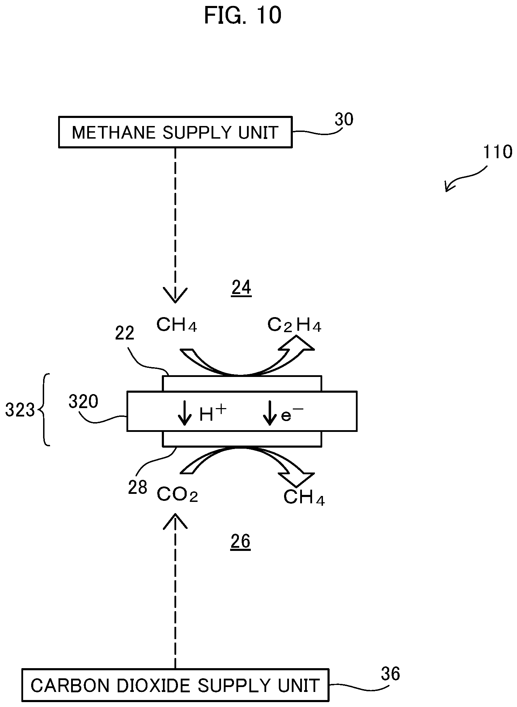

Fig. 10 is a view illustrating a schematic configuration of an ethylene production apparatus 110 of the tenth embodiment. The ethylene production apparatus 110 has the same structure as the ethylene production apparatus 106 of the sixth embodiment, except that the ceramic membrane 120 is replaced by a ceramic membrane 320, and the apparatus does not include the charge transfer unit 40 having the external circuit 42. The ceramic membrane 320 formed in the ethylene production apparatus 110 of the tenth embodiment is similar to the ceramic membrane 320 in the eighth embodiment.

In the ethylene production apparatus 110 of the tenth embodiment, reactions similar to those in the ethylene production apparatus 106 of the sixth embodiment proceed. Specifically, the reactions indicated in the formula (16) (same as the reaction of the formula (7)) and the formulas (10) to (12) proceed in the first catalyst layer 22, and the reaction shown in the formula (17) takes place in the second catalyst layer 28. The reaction indicated in the formula (18) proceeds in the whole of the ethylene production apparatus 108.

The ethylene production apparatus 110 of the tenth embodiment differs from the ethylene production apparatus 106 of the sixth embodiment in that the electrons generated in the reaction of the formula (16) are transferred to the second catalyst layer 28 through the inside of the ceramic membrane 320 and participate in the reaction of the formula (17). Thus, the ceramic membrane 320 in the tenth embodiment functions as a charge transfer unit.

Similarly to the case of the ethylene production apparatus 106 of the sixth embodiment, the above configuration can effectively enhance the ethylene production efficiency. Furthermore, the ethylene production apparatus 110 of the tenth embodiment generates methane in the second catalyst layer 28 along with the ethylene formation in the first catalyst layer 22. The methane obtained may be used for energy production or may be used as a material for synthesis of other compounds. Furthermore, the ceramic membrane 320 constructs a charge transfer unit and eliminates the need of the external circuit 42, thereby simplifying the configuration of the apparatus.

K. Eleventh embodiment:

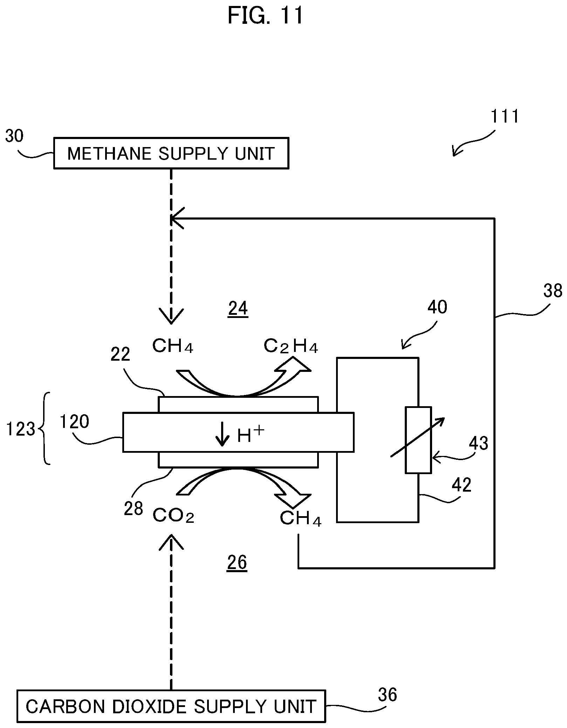

Fig. 11 is a view illustrating a schematic configuration of an ethylene production apparatus 111 of the eleventh embodiment. The ethylene production apparatus 111 has the same structure as the ethylene production apparatus 106 of the sixth embodiment, except that the apparatus further includes a methane recovery unit 38.

In the ethylene production apparatus 111 of the eleventh embodiment, reactions similar to those in the ethylene production apparatus 106 of the sixth embodiment proceed. Specifically, the reactions indicated in the formula (16) (same as the reaction of the formula (7)) and the formulas (10) to (12) proceed in the first catalyst layer 22, and the reaction shown in the formula (17) takes place in the second catalyst layer 28. The reaction indicated in the formula (18) proceeds in the whole of the ethylene production apparatus 108.

The methane recovery unit 38 present in the ethylene production apparatus 111 mixes methane produced in the second catalyst layer 28 on the second face of the ceramic membrane 120 to the methane-containing gas being supplied from the methane supply unit 30 to the first space 24. Specifically, the methane recovery unit 38 in the eleventh embodiment is a channel that connects an outlet from which methane is discharged from the second space 26 in the ethylene production apparatus 111 to a channel that connects the methane supply unit 30 to the first space 24 and through which the methane-containing gas flows.

Similarly to the case of the ethylene production apparatus 106 of the sixth embodiment, the above configuration can effectively enhance the ethylene production efficiency. Furthermore, the ethylene production apparatus 111 of the eleventh embodiment guides methane generated in the second catalyst layer 28 along with the ethylene formation in the first catalyst layer 22, to the first space 24 through the methane recovery unit 38 and uses the methane for ethylene formation. Thus, the amount of methane to be supplied from the methane supply unit 30 for ethylene production can be saved compared to when there is no methane recovery unit 38, and the methane use efficiency can be enhanced.

While the ethylene production apparatus 111 of the eleventh embodiment is the addition of the methane recovery unit 38 to the ethylene production apparatus 106 of the sixth embodiment, the configuration may be altered. For example, a methane recovery unit 38 similar to that of the eleventh embodiment may be provided in the ethylene production apparatus 110 of the tenth embodiment. In this case too, methane produced in the second catalyst layer 28 is recovered and fed to the first space 24, and the similar effects can be obtained in saving the amount of methane to be supplied from the methane supply unit 30 compared to when there is no methane recovery unit 38 and in enhancing the methane use efficiency.

L. Other embodiments:

Among the embodiments described above, the ethylene production apparatuses of the first to the sixth embodiments are such that the charge transfer unit 40 transfers an electron from the first face to the second face of the ceramic membrane through the external circuit 42 by utilizing an electric motive force stemming from an electric potential difference between the first face and the second face of the ceramic membrane. This configuration may be altered. For example, the charge transfer unit 40 may be such that an external power supply instead of the load unit 43 is connected to the external circuit 42. Specifically, for example, the ethylene production apparatuses illustrated in Figs. 1 to 6 and Fig. 11 may be configured so that an external power supply is provided in place of the load unit 43 and an electron is transferred from the first face to the second face of the ceramic membrane through the external circuit 42 by utilizing an electric motive force from the external power supply.

While the above embodiments illustrate the ceramic membrane as an independent membrane as shown in Figs. 1 to 11, this configuration may be altered. For example, the ceramic membrane may be a membrane formed on a face of a porous carrier (substrate, support). In this case, the ceramic membrane may be formed as a gas-impermeable dense membrane on a porous carrier by, for example, a PVD method, such as a PLD method, a dipping method, a thermal spraying method, a sputtering method, or the like.

EXAMPLES

Hereinafter, the present disclosure will be described in greater detail based on examples. However, the present disclosure is not limited to the description of those examples.

(Evaluation of ethylene production apparatuses)

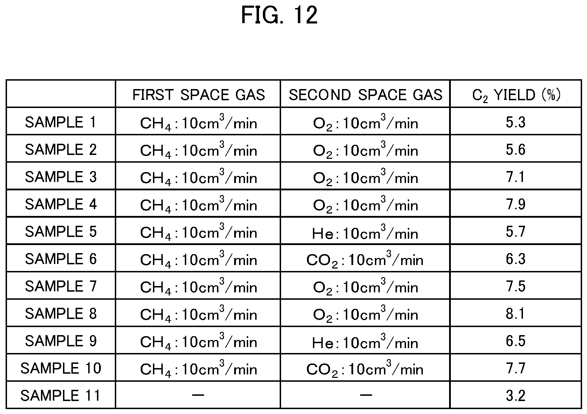

Fig. 12 is a view describing results of studying the performance of eleven types of ethylene production apparatuses representing Sample 1 to Sample 11. The configurations and the production methods of Samples, and the performance evaluation results will be described below. Samples 1 to 10 have configurations corresponding to the ethylene production apparatuses of the first to the tenth embodiments, respectively. Sample 11 is a comparative example.

<Production of membrane structures in Samples>

[Sample 1]

As illustrated in Fig. 1, the ethylene production apparatus of Sample 1 includes a membrane structure 21 that includes a ceramic membrane 20 having oxide ion conductivity and a first catalyst layer 22 disposed on a first face of the ceramic membrane 20.

The ceramic membrane 20 of Sample 1 was produced using yttria-stabilized zirconia (YSZ) as an oxide ion conductor. Specifically, TZ-8YS manufactured by Tosoh Corporation was used. A powder of YSZ was pressed into a disk shape with a hydraulic press machine and was sintered at 1400°C for 6 hours. The resultant sintered body was ground to a thickness of 0.15 mm with a surface grinder. The ceramic membrane 20 was thus obtained.

The first catalyst layer 22 of Sample 1 was produced using $BaZr_{0.4}Sc_{0.6}O_3$ as a catalyst for accelerating the synthesis of ethylene from methane. $BaZr_{0.4}Sc_{0.6}O_3$ was prepared by a complex polymerization method as follows. Barium nitrate (manufactured by FUJIFILM Wako Pure Chemical Corporation), zirconyl nitrate (manufactured by FUJIFILM Wako Pure Chemical Corporation), and scandium nitrate (manufactured by Alfa Aesar) were used as raw material powders. These raw material powders were weighed so that the ratio of the metal elements would correspond to the compositional ratio in the compositional formula $BaZr_{0.4}Sc_{0.6}O_3$. Furthermore, an aqueous citric acid solution and propylene glycol were added to the raw material powders so that the metal elements:citric acid:propylene glycol ratio = 1:3:3 (by mol). The mixture was stirred and mixed at 80°C for 1 hour. Furthermore, the solution was slowly heated to 300°C. A polymer in which the metal elements were dispersed was thus obtained. Furthermore, the polymer obtained was carbonized by heat treatment at 400°C for 2 hours. The carbonized product was crushed in an agate mortar to give a catalyst powder precursor. The catalyst powder precursor obtained was heat-treated at 1000°C for 6 hours. A catalyst powder 1 was thus obtained.

An ethyl cellulose binder and $\alpha$-terpineol solvent were added to the catalyst powder 1 obtained above, and the mixture was kneaded with a three-roll mill to give a catalyst paste 1. The catalyst paste 1 was applied to the first face of the ceramic membrane 20 by 10 mm-opening screen printing, and the coating was baked at 1000°C for 1 hour to form the first catalyst layer 22. The membrane structure 21 of Sample 1 was thus produced.

[Sample 2]

As illustrated in Fig. 2, the ethylene production apparatus of Sample 2 includes a membrane structure 121 that includes a ceramic membrane 120 having proton conductivity and a first catalyst layer 22 disposed on a first face of the ceramic membrane 120.

The ceramic membrane 120 of Sample 2 was produced using $BaZr_{0.8}Y_{0.2}O_3$ as a proton conductor. $BaZr_{0.8}Y_{0.2}O_3$ was prepared by a solid-phase reaction method as follows. Barium carbonate (manufactured by SAKAI CHEMICAL INDUSTRY CO.,LTD.), zirconium oxide (manufactured by DAIICHI KIGENSO KAGAKU KOGYO CO., LTD.), and yttrium oxide (manufactured by Shin-Etsu Chemical Co., Ltd.) were used as raw material powders. These raw material powders were weighed so that the ratio of the metal elements would correspond to the compositional ratio in the compositional formula $BaZr_{0.8}Y_{0.2}O_3$. The raw material powders were crushed and mixed in ethanol using $ZrO_2$ balls (manufactured by Nikkato Corporation) for 15 hours to give a slurry. The slurry obtained was dried in a hot water bath to give a mixed powder. The mixed powder obtained was calcined at 1300°C for 6 hours to give a $BaZr_{0.8}Y_{0.2}O_3$ powder. The powder obtained was pressed into a disk shape with a hydraulic press machine and was sintered at 1600°C for 10 hours. The resultant sintered body was ground to a thickness of 0.15 mm with a surface grinder. The ceramic membrane 120 was thus obtained.

The first catalyst layer 22 was formed on the first face of the ceramic membrane 120 in the same manner as in Sample 1. The membrane structure 121 of Sample 2 was thus produced.

[Sample 3]

As illustrated in Fig. 3, the ethylene production apparatus of Sample 3 includes a membrane structure 23 that includes a ceramic membrane 20 having oxide ion conductivity, a first catalyst layer 22 disposed on a first face of the ceramic membrane 20, and a second catalyst layer 28 disposed on a second face of the ceramic membrane 20.

The second catalyst layer 28 of Sample 3 was produced using $La_{0.6}Sr_{0.4}Co_{0.2}Fe_{0.8}O_3$ as a catalyst for accelerating the reaction of the formula (2). $La_{0.6}Sr_{0.4}Co_{0.2}Fe_{0.8}O_3$ was prepared by a solid-phase reaction method as follows.

Lanthanum oxide (manufactured by FUJIFILM Wako Pure Chemical Corporation), strontium carbonate (manufactured by Kojundo Chemical Lab. Co., Ltd.), cobalt oxide (manufactured by Kojundo Chemical Lab. Co., Ltd.), and iron oxide (manufactured by Kojundo Chemical Lab. Co., Ltd.) were used as raw material powders. A catalyst paste 2 was prepared using the above raw material powders in the same manner as the catalyst paste 1 for the first catalyst layer 22 described in Sample 1. Using the catalyst paste 2, the second catalyst layer 28 was formed in the same manner as the first catalyst layer 22, on the second face of a membrane structure similar to the membrane structure 21 of Sample 1. The membrane structure 23 of Sample 3 was thus produced.

[Sample 4]

As illustrated in Fig. 4, the ethylene production apparatus of Sample 4 includes a membrane structure 123 that includes a ceramic membrane 120 having proton conductivity, a first catalyst layer 22 disposed on a first face of the ceramic membrane 120, and a second catalyst layer 28 disposed on a second face of the ceramic membrane 120.
Similarly to the second catalyst layer 28 of Sample 3, the second catalyst layer 28 of Sample 4 was produced using $La_{0.6}Sr_{0.4}Co_{0.2}Fe_{0.8}O_3$ as a catalyst for accelerating the reaction of the formula (8). The second catalyst layer 28 was formed in the same manner as the second catalyst layer 28 of Sample 3, on the second face of a membrane structure similar to the membrane structure 121 of Sample 2. The membrane structure 123 of Sample 4 was thus produced.

[Sample 5]

As illustrated in Fig. 5, the ethylene production apparatus of Sample 5 includes a membrane structure 123 that includes a ceramic membrane 120 having proton conductivity, a first catalyst layer 22 disposed on a first face of the ceramic membrane 120, and a second catalyst layer 28 disposed on a second face of the ceramic membrane 120.
Similarly to the second catalyst layers 28 of Sample 3 and Sample 4, the second catalyst layer 28 of Sample 5 was produced using $La_{0.6}Sr_{0.4}Co_{0.2}Fe_{0.8}O_3$ as a catalyst for accelerating the reaction of the formula (14). That is, the membrane structure 123 of Sample 5 has the same configuration as the membrane structure 123 of Sample 4.

[Sample 6]

As illustrated in Fig. 6, the ethylene production apparatus of Sample 6 includes a membrane structure 123 that includes a ceramic membrane 120 having proton conductivity, a first catalyst layer 22 disposed on a first face of the ceramic membrane 120, and a second catalyst layer 28 disposed on a second face of the ceramic membrane 120.
The second catalyst layer 28 of Sample 6 was produced using nickel as a catalyst for accelerating the reaction of the formula (17). The second catalyst layer 28 was produced as follows. A catalyst paste 2 of Sample 6 was prepared using nickel oxide (manufactured by Kojundo Chemical Lab. Co., Ltd.) as a raw material powder in the same manner as the catalyst paste 2 of Sample 3. Using the catalyst paste 2, the second catalyst layer 28 was formed in the same manner as the second catalyst layer 28 of Sample 3, on the second face of a membrane structure similar to the membrane structure 121 of Sample 2. The membrane structure 123 of Sample 6 was thus produced.

[Sample 7]

As illustrated in Fig. 7, the ethylene production apparatus of Sample 7 includes a membrane structure 223 that includes a ceramic membrane 220 having oxide ion conductivity and electron conductivity, a first catalyst layer 22 disposed on a first face of the ceramic membrane 220, and a second catalyst layer 28 disposed on a second face of the ceramic membrane 220.
The ceramic membrane 220 of Sample 7 was produced by mixing an oxide ion conductor and an electron conductor. The oxide ion conductor that was used was yttria-stabilized zirconia (YSZ) similarly to the ceramic membrane 20 of Sample 1. $La_{0.8}Sr_{0.2}CrO_3$ was used as the electron conductor. $La_{0.8}Sr_{0.2}CrO_3$ was prepared by the solid-phase reaction method described hereinabove using lanthanum oxide (manufactured by FUJIFILM Wako Pure Chemical Corporation), strontium carbonate (manufactured by Kojundo Chemical Lab. Co., Ltd.), and chromium oxide (manufactured by Kojundo Chemical Lab. Co., Ltd.) as raw material powders.
A powder of yttria-stabilized zirconia (YSZ) and a powder of $La_{0.8}Sr_{0.2}CrO_3$ were weighed so that the volume ratio would be YSZ:$La_{0.8}Sr_{0.2}CrO_3$ = 70:30. These powders were crushed and mixed in ethanol using $ZrO_2$ balls (manufactured by Nikkato Corporation) for 15 hours to give a slurry. The slurry obtained was dried in a hot water bath to give a mixed powder. The mixed powder obtained was pressed into a disk shape with a hydraulic press machine and was sintered at 1500°C for 12 hours. The resultant sintered body was ground to a thickness of 0.15 mm with a surface grinder. The ceramic membrane 220 was thus obtained. The first catalyst layer 22 described hereinabove was formed on the first face of the ceramic membrane 220, and the second catalyst layer 28 similar to the second catalyst layer 28 of Sample 3 was formed on the second face of the ceramic membrane 220. The membrane structure 223 of Sample 7 was thus produced.

[Sample 8]

As illustrated in Fig. 8, the ethylene production apparatus of Sample 8 includes a membrane structure 323 that includes a ceramic membrane 320 having proton conductivity and electron conductivity, a first catalyst layer 22 disposed on a first face of the ceramic membrane 320, and a second catalyst layer 28 disposed on a second face of the ceramic membrane 320.

The ceramic membrane 320 of Sample 8 was produced by mixing a proton conductor and an electron conductor. The proton conductor that was used was $BaZr_{0.8}Y_{0.2}O_3$ similarly to the ceramic membrane 120 of Sample 2. The electron conductor that was used was $La_{0.8}Sr_{0.2}CrO_3$ similarly to the ceramic membrane 220 of Sample 7.

A powder of $BaZr_{0.8}Y_{0.2}O_3$ and a powder of $La_{0.8}Sr_{0.2}CrO_3$ were weighed so that the volume ratio would be $BaZr_{0.8}Y_{0.2}O_3:La_{0.8}Sr_{0.2}CrO_3 = 70:30$. A sintered body was obtained and ground in the same manner as in Sample 7. The ceramic membrane 320 was thus obtained. The first catalyst layer 22 described hereinabove was formed on the first face of the ceramic membrane 320, and the second catalyst layer 28 similar to the second catalyst layer 28 of Sample 4 was formed on the second face of the ceramic membrane 320. The membrane structure 323 of Sample 8 was thus produced.

[Sample 9]

As illustrated in Fig. 9, the ethylene production apparatus of Sample 9 includes a membrane structure 323 that includes a ceramic membrane 320 having proton conductivity and electron conductivity, a first catalyst layer 22 disposed on a first face of the ceramic membrane 320, and a second catalyst layer 28 disposed on a second face of the ceramic membrane 320.

The ceramic membrane 320 of Sample 9 was produced in the same manner as the ceramic membrane 320 of Sample 8. The first catalyst layer 22 described hereinabove was formed on the first face of the ceramic membrane 320, and the second catalyst layer 28 similar to the second catalyst layer 28 of Sample 5 was formed on the second face of the ceramic membrane 320. The membrane structure 323 of Sample 9 was thus produced.

[Sample 10]

As illustrated in Fig. 10, the ethylene production apparatus of Sample 10 includes a membrane structure 323 that includes a ceramic membrane 320 having proton conductivity and electron conductivity, a first catalyst layer 22 disposed on a first face of the ceramic membrane 320, and a second catalyst layer 28 disposed on a second face of the ceramic membrane 320.

The ceramic membrane 320 of Sample 10 was produced in the same manner as the ceramic membranes 320 of Sample 8 and Sample 9. The first catalyst layer 22 described hereinabove was formed on the first face of the ceramic membrane 320, and the second catalyst layer 28 similar to the second catalyst layer 28 of Sample 6 was formed on the second face of the ceramic membrane 320. The membrane structure 323 of Sample 10 was thus produced.

[Sample 11]

The ethylene production apparatus of Sample 11 has no ceramic membranes and uses only an oxidative-coupling-of-methane catalyst. The oxidative-coupling-of-methane catalyst used in Sample 11 was the catalyst powder 1 prepared to form the first catalyst layer 22 in Sample 1.

<Measurement of $C_2$ yields of Samples 1 to 10>

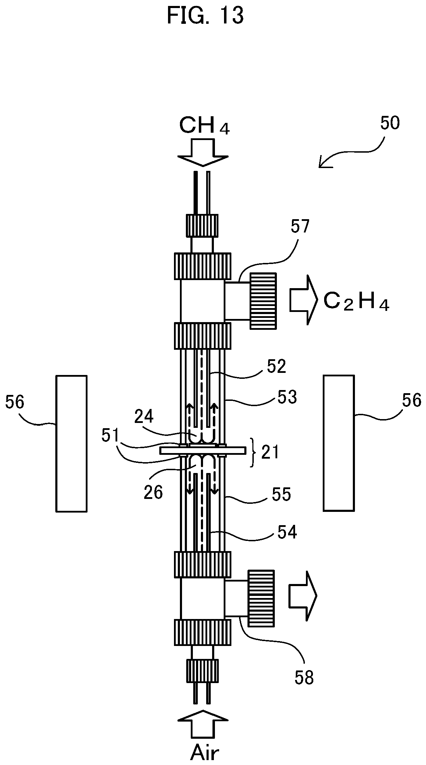

Fig. 13 is a view illustrating a schematic configuration of a measurement apparatus 50 for measuring the $C_2$ yield in the ethylene production apparatuses of Samples 1 to 10. Fig. 13 illustrates the measurement with the membrane structure 21 of Sample 1 as an example. The configuration of the measurement apparatus also applies to other Samples.

The measurement apparatus 50 includes two outer alumina tubes 53 and 55, two inner alumina tubes 52 and 54, and an electric furnace 56. The two outer alumina tubes 53 and 55 are arranged one above the other, and the membrane structure of Sample to be measured is sandwiched therebetween. The outer alumina tubes 53 and 55 and the membrane structure were joined in such a manner that thin rings 51 of gold having an inner diameter of 10 mm were placed on both faces of the ceramic membrane exposed at the outer periphery of the membrane structure without being covered with the catalyst layer; the outer alumina tubes 53 and 55 were pressed against the ceramic membrane via the thin rings 51; and the temperature was raised to 1050°C to soften the gold, thereby ensuring gas sealability. The inner alumina tube 52 was arranged inside the outer alumina tube 53, and the inner alumina tube 54 was arranged inside the outer alumina tube 55. The space above the membrane structure in the outer alumina tube 53 is the first space 24, and the space above the membrane structure in the outer alumina tube 55 is the second space 26. The outer alumina tubes 53 and 55 fitted with the membrane structure were arranged within the electric furnace 56 so that the membrane structure would be located at the soaking zone of the electric furnace 56.

In each of the measurement apparatuses 50 for Samples assembled as described above, methane was supplied to the first space 24 through the inner alumina tube 52 at a flow rate of 10 $cm^3$/min. The gas containing ethylene that was generated by the oxidative-coupling-of-methane reaction in the first catalyst layer 22 was discharged from the first space 24 through a first discharge channel 57 via the flow path between the inner alumina tube 52 and the outer alumina tube 53. The gas described in Fig. 12 was supplied to the second space 26 through the inner alumina tube 54. The gas after the reaction on the face of the membrane structure exposed in the second space 26 was discharged from the second space 26 through a second discharge channel 58 via the flow path between the inner alumina tube 54 and the outer alumina tube 55.

The temperature of the electric furnace 56 was set to 700°C. Although not shown in Fig. 13, the measurement apparatuses 50 for Samples 1 to 6 were provided with an external circuit 42, and the current density was controlled to 0.25 A/$cm^2$ with a DC electronic load device (FK-160L2Z, manufactured by TAKASAGO, LTD.). The gas discharged from the first space 24 and the gas discharged from the second space 26 were analyzed by micro gas chromatography (3000A, manufactured by Agilent Technologies Japan, Ltd.) to determine the $C_2$ yield. The $C_2$ yields obtained are described in Fig. 12. Incidentally, the gas discharged from the first space 24 contains ethylene and also ethane generated as shown in the formula (5) or (11), but usually the reaction further proceeds as shown by the formula (6) or (12). Thus, most of the $C_2$ hydrocarbons obtained is ethylene (data not shown).

<Measurement of $C_2$ yield of Sample 11>

For the ethylene production apparatus of Sample 11, 0.1 g of the catalyst powder 1 described in Sample 1 was weighed and placed in a fixed-bed flow reactor, and a mixed gas of methane, oxygen, and nitrogen was flowed in a flow rate ratio of $CH_4$:$O_2$:$N_2$ = 3.8:1:4 at an elevated temperature of 700°C and at 1 atm and 45 $cm^3$/min. The catalytic activity was thereby tested. The compositions of the gas that was introduced into the reactor, and of the gas that was discharged were analyzed by micro gas chromatography (3000A, manufactured by Agilent Technologies Japan, Ltd.). The $C_2$ yield obtained as a result of the composition analysis is described Fig. 12.

As described in Fig. 12, the ethylene production apparatuses of Samples 1 to 10 that included the ceramic membrane 20, the first catalyst layer 22, and the charge transfer unit 40 exhibited higher $C_2$ yields than the ethylene production apparatus of Sample 11 tested with a fixed-bed flow reactor. In particular, it has been confirmed that the $C_2$ yield is enhanced by further providing the second catalyst layer 28, as can be seen from the comparison of Sample 1 and Sample 3 or the comparison of Sample 2 and Sample 4.

(Evaluation of oxidative-coupling-of-methane catalysts)

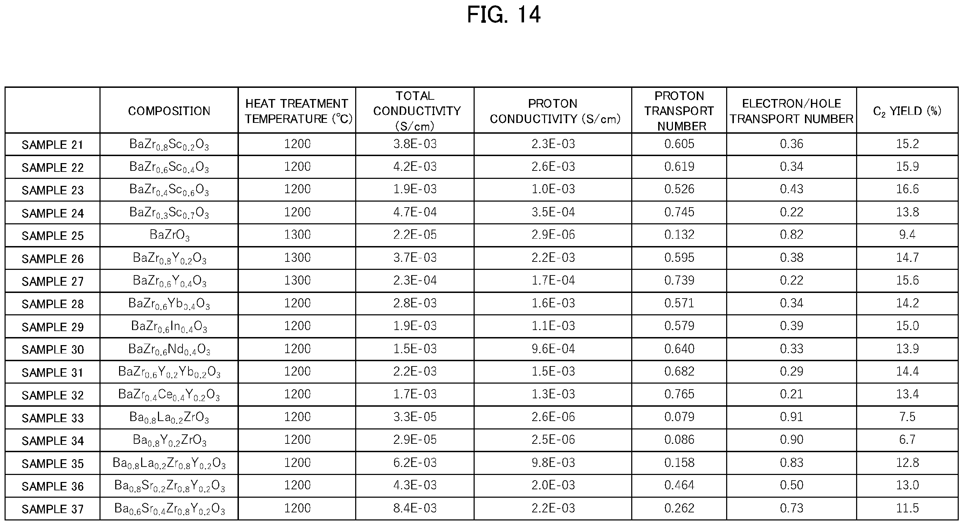

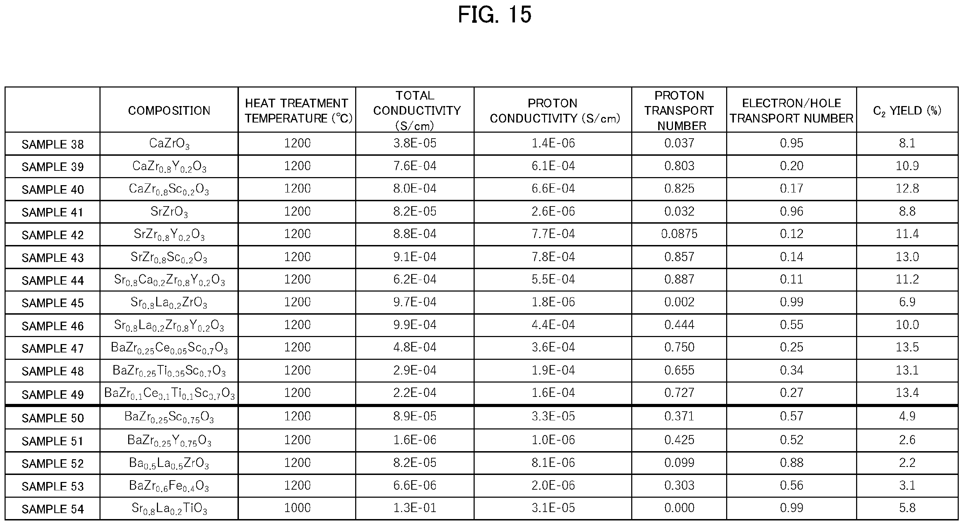

Figs. 14 and 15 are views describing results of studying the performance of 34 types of oxidative-coupling-of-methane catalysts of Samples 21 to 54. The configurations and the production methods of Samples, and the performance evaluation results will be described below.

<Production of Samples>

[Sample 21]

The catalyst of Sample 21 ($BaZr_{0.8}Sc_{0.2}O_3$) was prepared by a complex polymerization method. Barium nitrate (manufactured by FUJIFILM Wako Pure Chemical Corporation), zirconyl nitrate (manufactured by FUJIFILM Wako Pure Chemical Corporation), and scandium nitrate (manufactured by Alfa Aesar) were used as raw material powders. These raw material powders were weighed so that the ratio of the metal elements would correspond to the compositional ratio in the compositional formula $BaZr_{0.8}Sc_{0.2}O_3$. An aqueous citric acid solution and propylene glycol were added to the raw material powders so that the metal elements:citric acid:propylene glycol = 1:3:3 (by mol). The mixture was stirred and mixed at 80°C for 1 hour. Furthermore, the solution was slowly heated to 300°C. A polymer in which the metal elements were dispersed was thus obtained. Furthermore, the polymer obtained was carbonized by heat treatment at 400°C for 2 hours. The carbonized product was crushed in an agate mortar to give a catalyst powder precursor. The catalyst powder precursor obtained was heat-treated at 1200°C for 6 hours. A catalyst powder 21 was thus obtained.

[Samples 22 to 54]

Catalyst powders 22 to 54 were obtained in the same manner as in Sample 21, except that the types and the amounts of the raw material powders for the catalyst were changed so that the compositional ratios would be as described in the compositional formulas of Samples 22 to 54 in Figs. 14 and 15, and the temperature of the heat treatment of the catalyst powder precursor was changed as described in Figs. 14 and 15. Barium, zirconium, scandium, yttrium, ytterbium, indium, neodymium, cerium, lanthanum, strontium, calcium, iron, and titanium as constituent elements were added in the forms of raw material powders of barium nitrate (manufactured by FUJIFILM Wako Pure Chemical Corporation), zirconyl nitrate (manufactured by FUJIFILM Wako Pure Chemical Corporation), scandium nitrate (manufactured by Alfa Aesar), yttrium nitrate (manufactured by Sigma-Aldrich), ytterbium nitrate (manufactured by Sigma-Aldrich), indium nitrate (manufactured by FUJIFILM Wako Pure Chemical Corporation), neodymium nitrate (manufactured by FUJIFILM Wako Pure Chemical Corporation), cerium nitrate (manufactured by FUJIFILM Wako Pure Chemical Corporation), lanthanum nitrate (manufactured by FUJIFILM Wako Pure Chemical Corporation), strontium nitrate (manufactured by FUJIFILM Wako Pure Chemical Corporation), calcium nitrate (manufactured by FUJIFILM Wako Pure Chemical Corporation), iron nitrate (manufactured by Sigma-Aldrich), and tetra-i-propoxytitanium (manufactured by Kojundo Chemical Lab. Co., Ltd.) .

<Measurement of $C_2$ yields>

The catalyst powders 21 to 54 were weighed, each in an amount of 0.1 g, and were each placed in a fixed-bed flow reactor. A mixed gas of methane, oxygen, and nitrogen was flowed in a flow rate ratio of $CH_4:O_2:N_2 = 3.8:1:4$ at an elevated temperature of 750°C and at 1 atm and 45 $cm^3$/min. The catalytic activity was thereby tested. The compositions of the gas that was introduced into the reactor, and of the gas that was discharged were analyzed by micro gas chromatography (3000A, manufactured by Agilent Technologies Japan, Ltd.). The $C_2$ yields obtained as a result of the composition analysis are described in Figs. 14 and 15.

<Measurement of total conductivity>

The catalyst powders 21 to 54 were each pressed into a cuboid, which was then sintered at 1600°C for 6 hours. Platinum wires were wound at 4 locations, and a platinum paste (TR-7905, manufactured by Tanaka Kikinzoku Kogyo K.K.) was applied over the wires. Baking was performed at 1000°C for 1 hour. Measurement specimens were thus prepared. The measurement specimens were each placed in a tubular electric furnace and heated while flowing hydrogen humidified with a bubbler. The total conductivity was measured by an AC four-point probe method at 750°C.

<Measurement of proton transport number and electron/hole transport number>

The catalyst powders 21 to 54 were each pressed into a disk shape, which was then sintered at 1600°C for 6 hours. The platinum paste was applied by screen printing to draw circles on both faces of the disk. Baking was performed at 1000°C for 1 hour. Measurement specimens were thus prepared. The transport number measurement apparatus is equipped with two alumina tubes that are axially arranged one above the other. The measurement specimen was interposed between the two alumina tubes, and gas sealability was ensured between the alumina tubes and the measurement specimen. The portion including the measurement specimen was placed in the electric furnace, and the transport numbers were measured. Specifically, the temperature was increased to 750°C using the electric furnace while flowing humidified hydrogen in the respective spaces, the first space and the second space, within the two alumina tubes that were partitioned by the measurement specimen, in such a manner that the concentrations or the amounts of humidification were different between the spaces. The electric motive force generated between the first face and the second face of the measurement specimen was measured, and the proton transport number and the electron/hole transport number (the sum of the electron transport number and the hole transport number) were determined.

<Derivation of proton conductivity>

Proton conductivity was calculated by multiplying the total conductivity measured as described above by the proton transport number.

Among Samples described in Figs. 14 and 15, the catalysts of Samples 21 to 49 satisfy the condition that the catalyst has a perovskite structure represented by general formula $(A_{1-x}A'_x)(Zr_{1-y-z}B_yB'_z)O_3$ (wherein A is at least one element selected from alkaline earth metals, A' is at least one element of lanthanum (La) and yttrium (Y), B is at least one element of titanium (Ti) and cerium (Ce), B' is at least one element selected from yttrium (Y), scandium (Sc), ytterbium (Yb), aluminum (Al), indium (In), and neodymium (Nd), and the letters x, y, and z satisfy:

$$0 \leq x \leq 0.4,$$

$$0.3 \leq (1 - z) \leq 1,$$

$$0 \leq y,$$

and

$$0 < (1 - y - z)).$$

As described in Figs. 14 and 15, the catalysts of Samples 21 to 49 achieved a higher $C_2$ yield than the catalysts of Samples 50 to 54 as comparative examples that did not satisfy the above condition. Thus, the catalyst satisfying the above condition will offer an improvement in the performance of the ethylene production apparatus when applied to the first catalyst layer 22 in the ethylene production apparatuses of the first to the eleventh embodiments.

Among the catalysts of Samples 21 to 49, Samples 21 to 24, 26 to 32, 35 to 37, 39, 40, 42 to 44, and 46 to 49 exhibited a relatively high value of proton conductivity of $1.0 \times 10^{-4}$ S/cm or more. Among the catalysts of Samples 21 to 49, Samples 21 to 44 and 46 to 49 had a proton transport number of 0.01 or more. Among the catalysts of Samples 21 to 49, Samples 21 to 40, 42 to 44, and 46 to 49 had a sum of the electron transport number and the hole transport number of 0.01 or more and 0.95 or less. Among the catalysts of Samples 21 to 49, Samples 21 to 32, 35 to 37, 39, 40, 43, 44, and 46 to 49 had a proton transport number of 0.10 or more and a sum of the electron transport number and the hole transport number of 0.10 or more. These catalysts will offer a further improvement in the performance of the ethylene production apparatus when applied to the first catalyst layer 22 in the ethylene production apparatuses according to the embodiments in which the first catalyst layer 22 catalyzes the transfer of protons or electrons.

The present disclosure is not limited to the embodiments and other examples discussed above and may be implemented with various configurations that do not depart from the scope of the present disclosure. For example, the technical features in the embodiments corresponding to the technical features according to the aspects described in the section of Summary of Invention may be replaced or combined appropriately in order to solve part or all of the problems discussed hereinabove, or to achieve part or all of the advantageous effects described hereinabove. Furthermore, technical features that are not described as essential in the description may be deleted as appropriate.

Reference Signs List

| | |
|---|---|
| 20, 120, 220, 320 | ceramic membrane |
| 21, 23, 121, 123, 223, 323 | membrane structure |
| 22 | first catalyst layer |
| 24 | first space |
| 26 | second space |
| 28 | second catalyst layer |
| 30 | methane supply unit |
| 32 | oxygen supply unit |
| 34 | inert gas supply unit |
| 36 | carbon dioxide supply unit |
| 38 | methane recovery unit |
| 40 | charge transfer unit |
| 42 | external circuit |
| 43 | load unit |
| 50 | measurement apparatus |
| 51 | thin ring |
| 52, 54 | inner alumina tube |
| 53, 55 | outer alumina tube |
| 56 | electric furnace |
| 57 | first discharge channel |

| 58 | second discharge channel |
| 101 to 111 | ethylene production apparatus |

## Claims

1. An ethylene production apparatus for producing ethylene from a methane-containing gas, comprising:

   a ceramic membrane having at least one of oxide ion conductivity and proton conductivity;
   a first catalyst layer that is disposed on a first face of the ceramic membrane and includes a catalyst for accelerating oxidative-coupling-of-methane reaction producing ethylene from methane;
   a methane supply unit that supplies the methane-containing gas to a space that is one of two spaces separated from each other by the ceramic membrane and is located on the same side as the first face of the ceramic membrane; and
   a charge transfer unit that transfers an electron from the first face to a second face of the ceramic membrane or transfers a hole from the second face to the first face of the ceramic membrane,
   the ethylene production apparatus producing ethylene in the first catalyst layer upon supply of the methane-containing gas from the methane supply unit to the space on the same side as the first face.

2. The ethylene production apparatus in accordance with claim 1, wherein

   the ethylene production apparatus further comprises an oxygen supply unit that supplies an oxygen-containing gas which contains oxygen to a space located on the same side as the second face of the ceramic membrane; and
   the charge transfer unit includes an external circuit that connects the first face and the second face of the ceramic membrane to each other, and the charge transfer unit transfers an electron from the first face to the second face of the ceramic membrane through the external circuit by utilizing an electric motive force stemming from an electric potential difference between the first face of the ceramic membrane and the second face of the ceramic membrane.

3. The ethylene production apparatus in accordance with claim 2, wherein

   the ceramic membrane has oxide ion conductivity; and
   the ethylene production apparatus further comprises a second catalyst layer that is disposed on the second face of the ceramic membrane and includes a catalyst for accelerating a reaction producing an oxide ion from oxygen.

4. The ethylene production apparatus in accordance with claim 2, wherein

   the ceramic membrane has proton conductivity; and
   the ethylene production apparatus further comprises a second catalyst layer that is disposed on the second face of the ceramic membrane and includes a catalyst for accelerating a reaction using oxygen and a proton.

5. The ethylene production apparatus in accordance with claim 1, wherein

   the ceramic membrane has proton conductivity;
   the ethylene production apparatus further comprises:

      an inert gas supply unit that supplies an inert gas to a space located on the same side as the second face of the ceramic membrane, or a decompression unit that reduces the pressure in a space located on the same side as the second face of the ceramic membrane, and
      a second catalyst layer that is disposed on the second face of the ceramic membrane and includes a catalyst for accelerating a reaction producing hydrogen from protons; and

   the charge transfer unit includes an external circuit that connects the first face and the second face of the ceramic membrane to each other, and the charge transfer unit transfers an electron from the first face to the second face of the ceramic membrane through the external circuit by utilizing an electric motive force stemming from an electric potential difference between the first face of the ceramic membrane and the second face of the ceramic membrane.

6. The ethylene production apparatus in accordance with claim 1, wherein

the ceramic membrane has proton conductivity;
the ethylene production apparatus further comprises:

a carbon dioxide supply unit that supplies a carbon dioxide-containing gas which contains carbon dioxide to a space located on the same side as the second face of the ceramic membrane, and
a second catalyst layer that is disposed on the second face of the ceramic membrane and includes a catalyst for accelerating a reaction producing methane from carbon dioxide and protons; and

the charge transfer unit includes an external circuit that connects the first face and the second face of the ceramic membrane to each other, and the charge transfer unit transfers an electron from the first face to the second face of the ceramic membrane through the external circuit by utilizing an electric motive force stemming from an electric potential difference between the first face of the ceramic membrane and the second face of the ceramic membrane.

7. The ethylene production apparatus in accordance with claim 1, wherein

the ceramic membrane has at least one of electron conductivity and hole conductivity in addition to at least one of oxide ion conductivity and proton conductivity; and
the charge transfer unit is the ceramic membrane and allows an electron to transfer from the first face to the second face or allows a hole to transfer from the second face to the first face through the inside of the ceramic membrane itself along with occurrence of ethylene on the first face of the ceramic membrane.

8. The ethylene production apparatus in accordance with claim 7, wherein

the ceramic membrane has oxide ion conductivity and at least one of electron conductivity and hole conductivity; and
the ethylene production apparatus further comprises:

an oxygen supply unit that supplies an oxygen-containing gas which contains oxygen to a space located on the same side as the second face of the ceramic membrane, and
a second catalyst layer that is disposed on the second face of the ceramic membrane and includes a catalyst for accelerating a reaction producing an oxide ion from oxygen.

9. The ethylene production apparatus in accordance with claim 7, wherein

the ceramic membrane has proton conductivity and at least one of electron conductivity and hole conductivity; and
the ethylene production apparatus further comprises:

an oxygen supply unit that supplies an oxygen-containing gas which contains oxygen to a space located on the same side as the second face of the ceramic membrane, and
a second catalyst layer that is disposed on the second face of the ceramic membrane and includes a catalyst for accelerating a reaction using oxygen and a proton.

10. The ethylene production apparatus in accordance with claim 7, wherein

the ceramic membrane has proton conductivity; and
the ethylene production apparatus further comprises:

an inert gas supply unit that supplies an inert gas to a space located on the same side as the second face of the ceramic membrane, or a decompression unit that reduces the pressure in a space located on the same side as the second face of the ceramic membrane, and
a second catalyst layer that is disposed on the second face of the ceramic membrane and includes a catalyst for accelerating a reaction producing hydrogen from protons.

11. The ethylene production apparatus in accordance with claim 7, wherein

the ceramic membrane has proton conductivity; and
the ethylene production apparatus further comprises:

a carbon dioxide supply unit that supplies a carbon dioxide-containing gas which contains carbon dioxide to a space located on the same side as the second face of the ceramic membrane, and
a second catalyst layer that is disposed on the second face of the ceramic membrane and includes a catalyst for accelerating a reaction producing methane from carbon dioxide and protons.

12. The ethylene production apparatus in accordance with claim 6 or 11, wherein
the ethylene production apparatus further comprises a methane recovery unit that mixes methane produced on the second face of the ceramic membrane to the methane-containing gas being supplied from the methane supply unit to the space on the same side as the first face of the ceramic membrane.

13. The ethylene production apparatus in accordance with any one of claims 1 to 12, wherein
the catalyst in the first catalyst layer has:
a perovskite structure represented by general formula $(A_{1-x}A'_x)(Zr_{1-y-z}B_yB'_z)O_3$ (wherein A is at least one element selected from alkaline earth metals, A' is at least one element of lanthanum (La) and yttrium (Y), B is at least one element of titanium (Ti) and cerium (Ce), B' is at least one element selected from yttrium (Y), scandium (Sc), ytterbium (Yb), aluminum (Al), indium (In), and neodymium (Nd), and the letters x, y, and z satisfy:

$$0 \leq x \leq 0.4,$$

$$0.3 \leq (1 - z) \leq 1,$$

$$0 \leq y,$$

and

$$0 < (1 - y - z)).$$

14. The ethylene production apparatus in accordance with claim 1, wherein
the charge transfer unit includes an external circuit that connects the first face and the second face of the ceramic membrane to each other, and an external power supply connected to the external circuit, and the charge transfer unit transfers an electron from the first face to the second face of the ceramic membrane through the external circuit by utilizing an electric motive force from the external power supply.

15. An ethylene production method for producing ethylene from a methane-containing gas, comprising:

supplying the methane-containing gas to a space that is one of two spaces separated from each other by a ceramic membrane having at least one of oxide ion conductivity and proton conductivity and is located on the same side as a first face of the ceramic membrane, the first face bearing a first catalyst layer including a catalyst for accelerating oxidative-coupling-of-methane reaction producing ethylene from methane;
performing charge transfer between the first face and a second face of the ceramic membrane by transferring an electron from the first face to the second face of the ceramic membrane or transferring a hole from the second face to the first face of the ceramic membrane; and
producing ethylene from methane in the methane-containing gas on the first catalyst layer.

16. The ethylene production method in accordance with claim 15, wherein

the ethylene production method further comprises supplying an oxygen-containing gas which contains oxygen to a space located on the same side as the second face of the ceramic membrane; and
the charge transfer is performed by transferring an electron from the first face to the second face of the ceramic membrane through an external circuit that connects the first face and the second face of the ceramic membrane to each other, wherein the charge transfer utilizes an electric motive force stemming from an electric potential difference between the first face of the ceramic membrane and the second face of the ceramic membrane.

17. The ethylene production method in accordance with claim 16, wherein
the ceramic membrane is a membrane that has oxide ion conductivity and bears a second catalyst layer on the second face, the second catalyst layer including a catalyst for accelerating a reaction producing an oxide ion from oxygen.

18. The ethylene production method in accordance with claim 16, wherein
the ceramic membrane is a membrane that has proton conductivity and bears a second catalyst layer on the second face, the second catalyst layer including a catalyst for accelerating a reaction using oxygen and a proton.

19. The ethylene production method in accordance with claim 15, wherein

the ceramic membrane is a membrane that has proton conductivity and bears a second catalyst layer on the second face, the second catalyst layer including a catalyst for accelerating a reaction producing hydrogen from protons;
the ethylene production method further comprises supplying an inert gas to a space located on the same side as the second face of the ceramic membrane, or reducing the pressure in a space located on the same side as the second face of the ceramic membrane; and
the charge transfer is performed by transferring an electron from the first face to the second face of the ceramic membrane through an external circuit that connects the first face and the second face of the ceramic membrane to each other, wherein the charge transfer utilizes an electric motive force stemming from an electric potential difference between the first face of the ceramic membrane and the second face of the ceramic membrane.

20. The ethylene production method in accordance with claim 15, wherein

the ceramic membrane is a membrane that has proton conductivity and bears a second catalyst layer on the second face, the second catalyst layer including a catalyst for accelerating a reaction producing methane from carbon dioxide and protons;
the ethylene production method further comprises supplying a carbon dioxide-containing gas which contains carbon dioxide to a space located on the same side as the second face of the ceramic membrane; and
the charge transfer is performed by transferring an electron from the first face to the second face of the ceramic membrane through an external circuit that connects the first face and the second face of the ceramic membrane to each other, wherein the charge transfer utilizes an electric motive force stemming from an electric potential difference between the first face of the ceramic membrane and the second face of the ceramic membrane.

21. The ethylene production method in accordance with claim 15, wherein

the ceramic membrane is a membrane that has at least one of electron conductivity and hole conductivity in addition to at least one of oxide ion conductivity and proton conductivity; and
the charge transfer is performed by transferring an electron from the first face to the second face or transferring a hole from the second face to the first face through the inside of the ceramic membrane along with occurrence of ethylene on the first face of the ceramic membrane.

22. The ethylene production method in accordance with claim 21, wherein

the ethylene production method further comprises supplying an oxygen-containing gas which contains oxygen to a space located on the same side as the second face of the ceramic membrane; and
the ceramic membrane is a membrane that has oxide ion conductivity and at least one of electron conductivity and hole conductivity and bears a second catalyst layer on the second face, the second catalyst layer including a catalyst for accelerating a reaction producing an oxide ion from oxygen.

23. The ethylene production method in accordance with claim 21, wherein

the ethylene production method further comprises supplying an oxygen-containing gas which contains oxygen to a space located on the same side as the second face of the ceramic membrane; and
the ceramic membrane is a membrane that has proton conductivity and at least one of electron conductivity and hole conductivity and bears a second catalyst layer on the second face, the second catalyst layer including a catalyst for accelerating a reaction using oxygen and a proton.

24. The ethylene production method in accordance with claim 21, wherein

the ethylene production method further comprises supplying an inert gas to a space located on the same side as the second face of the ceramic membrane, or reducing the pressure in a space located on the same side as the second face of the ceramic membrane; and
the ceramic membrane is a membrane that has proton conductivity and bears a second catalyst layer on the second face, the second catalyst layer including a catalyst for accelerating a reaction producing hydrogen from protons.

25. The ethylene production method in accordance with claim 21, wherein

the ethylene production method further comprises supplying a carbon dioxide-containing gas which contains carbon dioxide to a space located on the same side as the second face of the ceramic membrane; and
the ceramic membrane is a membrane that has proton conductivity and bears a second catalyst layer on the second face, the second catalyst layer including a catalyst for accelerating a reaction producing methane from carbon dioxide and protons.

26. The ethylene production method in accordance with claim 20 or 25, wherein
the ethylene production method further comprises mixing methane produced on the second face of the ceramic membrane to the methane-containing gas being supplied to the space on the same side as the first face of the ceramic membrane.

27. The ethylene production method in accordance with any one of claims 15 to 26, wherein
the catalyst in the first catalyst layer has:
a perovskite structure represented by general formula $(A_{1-x}A'_x)(Zr_{1-y-z}B_yB'_z)O_3$ (wherein A is at least one element selected from alkaline earth metals, A' is at least one element of lanthanum (La) and yttrium (Y), B is at least one element of titanium (Ti) and cerium (Ce), B' is at least one element selected from yttrium (Y), scandium (Sc), ytterbium (Yb), aluminum (Al), indium (In), and neodymium (Nd), and the letters x, y, and z satisfy:

$$0 \leq x \leq 0.4,$$

$$0.3 \leq (1 - z) \leq 1,$$

$$0 \leq y,$$

and

$$0 < (1 - y - z)).$$

28. The ethylene production method in accordance with claim 15, wherein the charge transfer is performed by transferring an electron from the first face to the second face of the ceramic membrane through an external circuit that connects the first face and the second face of the ceramic membrane to each other, wherein the charge transfer utilizes an electric motive force from an external power supply connected to the external circuit.

FIG. 1

METHANE SUPPLY UNIT — 30

101

24

22

CH$_4$    C$_2$H$_4$

21 {

↑ O$^{2-}$

20

O$_2$    26

40

43

42

OXYGEN SUPPLY UNIT — 32

# FIG. 2

METHANE SUPPLY UNIT — 30

102

24

CH$_4$     C$_2$H$_4$

22

121 {

↓ H$^+$

40

43

120

O$_2$     H$_2$O

42

26

OXYGEN SUPPLY UNIT — 32

# FIG. 3

METHANE SUPPLY UNIT — 30

103

$\underline{24}$

$CH_4$     $C_2H_4$

22

$23 \Big\{$ 20    $\uparrow O^{2-}$

28

40

43

42

$O_2$    $\underline{26}$

OXYGEN SUPPLY UNIT — 32

FIG. 4

METHANE SUPPLY UNIT — 30

104

24

$CH_4$        $C_2H_4$

22

$\downarrow H^+$

40

120

123 {

43

28

$O_2$

42

26

$H_2O$

OXYGEN SUPPLY UNIT — 32

# FIG. 5

# FIG. 6

METHANE SUPPLY UNIT ⟋ 30

106

<u>24</u>

CH₄          C₂H₄

22

123 { 120          ↓ H⁺

28 CO₂          CH₄

40

43

42

<u>26</u>

CARBON DIOXIDE SUPPLY UNIT ⟋ 36

# FIG. 7

METHANE SUPPLY UNIT — 30

107

24

$CH_4$    $C_2H_4$

22

223 { 220    $\uparrow O^{2-}$    $\downarrow e^-$

28

$O_2$    26

OXYGEN SUPPLY UNIT — 32

FIG. 8

METHANE SUPPLY UNIT — 30

108

24

CH$_4$   C$_2$H$_4$

22

323 { 320   $\downarrow$ H$^+$   $\downarrow$ e$^-$

28   O$_2$   H$_2$O

26

OXYGEN SUPPLY UNIT — 32

FIG. 9

METHANE SUPPLY UNIT — 30

109

24

CH₄      C₂H₄

22

323 { 320    ↓H⁺    ↓e⁻

28

26

H₂

He

INERT GAS SUPPLY UNIT — 34

# FIG. 10

METHANE SUPPLY UNIT — 30

110

<u>24</u>

CH$_4$    C$_2$H$_4$

22

323 {  320    ↓H$^+$  ↓e$^-$

28 CO$_2$    CH$_4$

<u>26</u>

CARBON DIOXIDE SUPPLY UNIT — 36

# FIG. 11

30 METHANE SUPPLY UNIT

111

24

22 CH₄    C₂H₄

123 { 120    ↓ H⁺

28 CO₂    CH₄

26

40

43

42

38

36 CARBON DIOXIDE SUPPLY UNIT

## FIG. 12

|  | FIRST SPACE GAS | SECOND SPACE GAS | $C_2$ YIELD (%) |
|---|---|---|---|
| SAMPLE 1 | $CH_4 : 10cm^3/min$ | $O_2 : 10cm^3/min$ | 5.3 |
| SAMPLE 2 | $CH_4 : 10cm^3/min$ | $O_2 : 10cm^3/min$ | 5.6 |
| SAMPLE 3 | $CH_4 : 10cm^3/min$ | $O_2 : 10cm^3/min$ | 7.1 |
| SAMPLE 4 | $CH_4 : 10cm^3/min$ | $O_2 : 10cm^3/min$ | 7.9 |
| SAMPLE 5 | $CH_4 : 10cm^3/min$ | $He : 10cm^3/min$ | 5.7 |
| SAMPLE 6 | $CH_4 : 10cm^3/min$ | $CO_2 : 10cm^3/min$ | 6.3 |
| SAMPLE 7 | $CH_4 : 10cm^3/min$ | $O_2 : 10cm^3/min$ | 7.5 |
| SAMPLE 8 | $CH_4 : 10cm^3/min$ | $O_2 : 10cm^3/min$ | 8.1 |
| SAMPLE 9 | $CH_4 : 10cm^3/min$ | $He : 10cm^3/min$ | 6.5 |
| SAMPLE 10 | $CH_4 : 10cm^3/min$ | $CO_2 : 10cm^3/min$ | 7.7 |
| SAMPLE 11 | — | — | 3.2 |

# FIG. 13

# FIG. 14

| | COMPOSITION | HEAT TREATMENT TEMPERATURE (°C) | TOTAL CONDUCTIVITY (S/cm) | PROTON CONDUCTIVITY (S/cm) | PROTON TRANSPORT NUMBER | ELECTRON/HOLE TRANSPORT NUMBER | $C_2$ YIELD (%) |
|---|---|---|---|---|---|---|---|
| SAMPLE 21 | $BaZr_{0.8}Sc_{0.2}O_3$ | 1200 | 3.8E-03 | 2.3E-03 | 0.605 | 0.36 | 15.2 |
| SAMPLE 22 | $BaZr_{0.6}Sc_{0.4}O_3$ | 1200 | 4.2E-03 | 2.6E-03 | 0.619 | 0.34 | 15.9 |
| SAMPLE 23 | $BaZr_{0.4}Sc_{0.6}O_3$ | 1200 | 1.9E-03 | 1.0E-03 | 0.526 | 0.43 | 16.6 |
| SAMPLE 24 | $BaZr_{0.3}Sc_{0.7}O_3$ | 1200 | 4.7E-04 | 3.5E-04 | 0.745 | 0.22 | 13.8 |
| SAMPLE 25 | $BaZrO_3$ | 1300 | 2.2E-05 | 2.9E-06 | 0.132 | 0.82 | 9.4 |
| SAMPLE 26 | $BaZr_{0.8}Y_{0.2}O_3$ | 1300 | 3.7E-03 | 2.2E-03 | 0.595 | 0.38 | 14.7 |
| SAMPLE 27 | $BaZr_{0.6}Y_{0.4}O_3$ | 1300 | 2.3E-04 | 1.7E-04 | 0.739 | 0.22 | 15.6 |
| SAMPLE 28 | $BaZr_{0.6}Yb_{0.4}O_3$ | 1200 | 2.8E-03 | 1.6E-03 | 0.571 | 0.34 | 14.2 |
| SAMPLE 29 | $BaZr_{0.6}In_{0.4}O_3$ | 1200 | 1.9E-03 | 1.1E-03 | 0.579 | 0.39 | 15.0 |
| SAMPLE 30 | $BaZr_{0.6}Nd_{0.4}O_3$ | 1200 | 1.5E-03 | 9.6E-04 | 0.640 | 0.33 | 13.9 |
| SAMPLE 31 | $BaZr_{0.6}Y_{0.2}Yb_{0.2}O_3$ | 1200 | 2.2E-03 | 1.5E-03 | 0.682 | 0.29 | 14.4 |
| SAMPLE 32 | $BaZr_{0.4}Ce_{0.4}Y_{0.2}O_3$ | 1200 | 1.7E-03 | 1.3E-03 | 0.765 | 0.21 | 13.4 |
| SAMPLE 33 | $Ba_{0.8}La_{0.2}ZrO_3$ | 1200 | 3.3E-05 | 2.6E-06 | 0.079 | 0.91 | 7.5 |
| SAMPLE 34 | $Ba_{0.8}Y_{0.2}ZrO_3$ | 1200 | 2.9E-05 | 2.5E-06 | 0.086 | 0.90 | 6.7 |
| SAMPLE 35 | $Ba_{0.8}La_{0.2}Zr_{0.8}Y_{0.2}O_3$ | 1200 | 6.2E-03 | 9.8E-03 | 0.158 | 0.83 | 12.8 |
| SAMPLE 36 | $Ba_{0.8}Sr_{0.2}Zr_{0.8}Y_{0.2}O_3$ | 1200 | 4.3E-03 | 2.0E-03 | 0.464 | 0.50 | 13.0 |
| SAMPLE 37 | $Ba_{0.6}Sr_{0.4}Zr_{0.8}Y_{0.2}O_3$ | 1200 | 8.4E-03 | 2.2E-03 | 0.262 | 0.73 | 11.5 |

FIG. 15

| | COMPOSITION | HEAT TREATMENT TEMPERATURE (°C) | TOTAL CONDUCTIVITY (S/cm) | PROTON CONDUCTIVITY (S/cm) | PROTON TRANSPORT NUMBER | ELECTRON/HOLE TRANSPORT NUMBER | $C_2$ YIELD (%) |
|---|---|---|---|---|---|---|---|
| SAMPLE 38 | $CaZrO_3$ | 1200 | 3.8E-05 | 1.4E-06 | 0.037 | 0.95 | 8.1 |
| SAMPLE 39 | $CaZr_{0.8}Y_{0.2}O_3$ | 1200 | 7.6E-04 | 6.1E-04 | 0.803 | 0.20 | 10.9 |
| SAMPLE 40 | $CaZr_{0.8}Sc_{0.2}O_3$ | 1200 | 8.0E-04 | 6.6E-04 | 0.825 | 0.17 | 12.8 |
| SAMPLE 41 | $SrZrO_3$ | 1200 | 8.2E-05 | 2.6E-06 | 0.032 | 0.96 | 8.8 |
| SAMPLE 42 | $SrZr_{0.8}Y_{0.2}O_3$ | 1200 | 8.8E-04 | 7.7E-04 | 0.0875 | 0.12 | 11.4 |
| SAMPLE 43 | $SrZr_{0.8}Sc_{0.2}O_3$ | 1200 | 9.1E-04 | 7.8E-04 | 0.857 | 0.14 | 13.0 |
| SAMPLE 44 | $Sr_{0.8}Ca_{0.2}Zr_{0.8}Y_{0.2}O_3$ | 1200 | 6.2E-04 | 5.5E-04 | 0.887 | 0.11 | 11.2 |
| SAMPLE 45 | $Sr_{0.8}La_{0.2}ZrO_3$ | 1200 | 9.7E-04 | 1.8E-06 | 0.002 | 0.99 | 6.9 |
| SAMPLE 46 | $Sr_{0.8}La_{0.2}Zr_{0.8}Y_{0.2}O_3$ | 1200 | 9.9E-04 | 4.4E-04 | 0.444 | 0.55 | 10.0 |
| SAMPLE 47 | $BaZr_{0.25}Ce_{0.05}Sc_{0.7}O_3$ | 1200 | 4.8E-04 | 3.6E-04 | 0.750 | 0.25 | 13.5 |
| SAMPLE 48 | $BaZr_{0.25}Ti_{0.05}Sc_{0.7}O_3$ | 1200 | 2.9E-04 | 1.9E-04 | 0.655 | 0.34 | 13.1 |
| SAMPLE 49 | $BaZr_{0.1}Ce_{0.1}Ti_{0.1}Sc_{0.7}O_3$ | 1200 | 2.2E-04 | 1.6E-04 | 0.727 | 0.27 | 13.4 |
| SAMPLE 50 | $BaZr_{0.25}Sc_{0.75}O_3$ | 1200 | 8.9E-05 | 3.3E-05 | 0.371 | 0.57 | 4.9 |
| SAMPLE 51 | $BaZr_{0.25}Y_{0.75}O_3$ | 1200 | 1.6E-06 | 1.0E-06 | 0.425 | 0.52 | 2.6 |
| SAMPLE 52 | $Ba_{0.5}La_{0.5}ZrO_3$ | 1200 | 8.2E-05 | 8.1E-06 | 0.099 | 0.88 | 2.2 |
| SAMPLE 53 | $BaZr_{0.6}Fe_{0.4}O_3$ | 1200 | 6.6E-06 | 2.0E-06 | 0.303 | 0.56 | 3.1 |
| SAMPLE 54 | $Sr_{0.8}La_{0.2}TiO_3$ | 1000 | 1.3E-01 | 3.1E-05 | 0.000 | 0.99 | 5.8 |

EP 4 361 122 A1

## INTERNATIONAL SEARCH REPORT

| International application No. |
|---|
| **PCT/JP2022/024127** |

**A. CLASSIFICATION OF SUBJECT MATTER**

*C07C 2/84*(2006.01)i; *B01J 23/02*(2006.01)i; *B01J 23/08*(2006.01)i; *B01J 23/10*(2006.01)i; *B01J 23/26*(2006.01)i; *B01J 23/755*(2006.01)i; *B01J 23/83*(2006.01)i; *B01J 23/86*(2006.01)i; *C07B 61/00*(2006.01)i; *C07C 2/80*(2006.01)i; *C07C 11/04*(2006.01)i; *C25B 3/03*(2021.01)i; *C25B 5/00*(2006.01)i; *C25B 9/00*(2021.01)i; *C25B 9/23*(2021.01)i; *C25B 11/052*(2021.01)i; *C25B 11/091*(2021.01)i; *H01M 8/00*(2016.01)i; *H01M 8/02*(2016.01)i

FI: C07C2/84; H01M8/02; H01M8/00 Z; C07C2/80; C07C11/04; C25B5/00; C25B9/00 H; C25B9/23; C25B11/052; C25B11/091; B01J23/10 Z; B01J23/755 Z; B01J23/26 Z; B01J23/86 Z; B01J23/83 Z; B01J23/02 Z; B01J23/08 Z; C25B3/03; C07B61/00 300

According to International Patent Classification (IPC) or to both national classification and IPC

**B. FIELDS SEARCHED**

Minimum documentation searched (classification system followed by classification symbols)

C07C2/84; B01J23/02; B01J23/08; B01J23/10; B01J23/26; B01J23/755; B01J23/83; B01J23/86; C07B61/00; C07C2/80; C07C11/04; C25B3/03; C25B5/00; C25B9/00; C25B9/23; C25B11/052; C25B11/091; H01M8/00; H01M8/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 6-056428 A (THE STANDARD OIL CO.) 01 March 1994 (1994-03-01) claim 13, page 11, left column, lines 15-25, page 12, right column, lines 16-18, page 14, left column, line 47 to right column, line 24 | 1-3, 7-8, 14-17, 21-22, 28 |
| Y | fig. 8, page 12, left column, line 42 to right column, line 18 | 2, 4-5, 13-14, 16, 18-19, 27-28 |
| X | US 6281403 B1 (ELTRON RESEARCH, INC.) 28 August 2001 (2001-08-28) claim 1, fig. 3, 6 | 1, 7, 9-10, 15, 21, 23-24 |
| Y | claim 1, fig. 3, 6 | 2, 4-6, 11-14, 16, 18-20, 25-28 |

☑ Further documents are listed in the continuation of Box C.　　☑ See patent family annex.

| * Special categories of cited documents: |
|---|

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 August 2022** | **30 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

## INTERNATIONAL SEARCH REPORT

| International application No. |
|---|
| **PCT/JP2022/024127** |

### C. DOCUMENTS CONSIDERED TO BE RELEVANT

| Category* | Citation of document, with indication, where appropriate, of the relevant passages | Relevant to claim No. |
|---|---|---|
| X | JP 6-088080 A (SUMITOMO CHEM. CO., LTD.) 29 March 1994 (1994-03-29) claims, examples 1-2, page 3, right column, lines 12-18 | 1, 7, 9, 15, 21, 23 |
| Y | claims, examples 1-2, page 3, right column, lines 12-18 | 2, 4, 13-14, 16, 18, 27-28 |
| X | JP 2-017947 A (ARUKO CHEM. CO.) 22 January 1990 (1990-01-22) claims 1, 10, example 5 | 1, 7, 15, 21 |
| Y | claims 1, 10, example 5 | 2, 13-14, 16, 27-28 |
| X | KR 10-2018-0065137 A (KOREA INSTITUTE OF ENERGY RESEARCH) 18 June 2018 (2018-06-18) claims 1-3, 10, fig. 1 | 1, 7, 15, 21 |
| Y | claims 1-3, 10, fig. 1 | 2, 13-14, 16, 27-28 |
| X | EP 2374526 A1 (CENTRE NATIONAL DE LA RECHERCHE SCIENTIFIQUE (C.N.R.S)) 12 October 2011 (2011-10-12) fig. 12, claims 1-2, paragraphs [0104], [0105] | 1, 7, 15, 21 |
| Y | fig. 12, claims 1-2, paragraphs [0104], [0105] | 2, 13-14, 16, 27-28 |
| Y | US 2018/0296974 A1 (UNIVERSITY OF MARYLAND, COLLEGE PARK) 18 October 2018 (2018-10-18) claims 1, 21-23 | 6, 11-13, 20, 25-27 |
| Y | US 2017/0267605 A1 (SILURIA TECHNOLOGIES, INC.) 21 September 2017 (2017-09-21) claims 11, 17, 27, example 4, paragraphs [0005], [0008] | 13, 27 |
| A | US 2008/0318094 A1 (GINOSAR, D. M.) 25 December 2008 (2008-12-25) entire text | 1-28 |

**INTERNATIONAL SEARCH REPORT**
Information on patent family members

| International application No. |
| --- |
| **PCT/JP2022/024127** |

| Patent document cited in search report | | | Publication date (day/month/year) | Patent family member(s) | | | Publication date (day/month/year) |
| --- | --- | --- | --- | --- | --- | --- | --- |
| JP | 6-056428 | A | 01 March 1994 | US | 2003/0155254 | A1 | |
| | | | | claim 23, fig. 8, paragraphs [0175], [0176], [0187], [0188], [0212]-[0215] | | | |
| | | | | EP | 766330 | A1 | |
| | | | | DE | 69030651 | C | |
| | | | | CN | 1055205 | A | |
| US | 6281403 | B1 | 28 August 2001 | (Family: none) | | | |
| JP | 6-088080 | A | 29 March 1994 | (Family: none) | | | |
| JP | 2-017947 | A | 22 January 1990 | US | 4791079 | A | |
| | | | | claim 1, example 5 | | | |
| | | | | EP | 345393 | A1 | |
| KR | 10-2018-0065137 | A | 18 June 2018 | (Family: none) | | | |
| EP | 2374526 | A1 | 12 October 2011 | (Family: none) | | | |
| US | 2018/0296974 | A1 | 18 October 2018 | WO | 2017/062663 | A1 | |
| US | 2017/0267605 | A1 | 21 September 2017 | WO | 2017/161171 | A2 | |
| | | | | EP | 3429747 | A2 | |
| US | 2008/0318094 | A1 | 25 December 2008 | WO | 2004/034487 | A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP H5238961 A

**Non-patent literature cited in the description**

• **BRITTANY LANCASTER FARRELL et al.** *ACS Catal.,* 2016, vol. 6, 4340-4346